(19) Europäisches Patentamt
European Patent Office
Office européen des brevets

(11) Numéro de publication: **0 107 570**
**B1**

(12) # FASCICULE DE BREVET EUROPÉEN

(45) Date de publication du fascicule du brevet: 15.01.86

(21) Numéro de dépôt: 83401977.0

(22) Date de dépôt: 11.10.83

(51) Int. Cl.⁴: **C 07 C 121/75**, C 07 C 69/743, C 07 C 69/708, C 07 C 147/00, C 07 C 149/06, C 07 D 213/64, C 07 D 233/74, A 01 N 53/00, A 23 K 1/16 // C07D307/93

(54) **Nouveaux dérivés de l'acide cyclopropane carboxylique substitués en 3 par une chaîne vinylique elle-même substituée, leur procédé et leurs intermédiaires de préparation, leur application comme pesticides et les compositions les renfermant.**

(30) Priorité: 12.10.82 FR 8217055

(43) Date de publication de la demande: 02.05.84 Bulletin 84/18

(45) Mention de la délivrance du brevet: 15.01.86 Bulletin 86/3

(84) Etats contractants désignés: BE CH DE FR GB IT LI LU NL

(56) Documents cité:
EP-A-0 050 534
DE-A-2 654 062
US-A-4 215 069

(73) Titulaire: ROUSSEL- UCLAF, 35, boulevard des Invalides, F-75007 Paris (FR)

(72) Inventeur: Tessier, Jean, 30, rue Jean Moulin, F-94300 Vincennes (FR)
Inventeur: Demoute, Jean- Pierre, 249 bis, rue de Rosny, F-93100 Montreuil- sous- Bois (FR)
Inventeur: Cadiergue, Joseph, 6, rue Jules Princet, F-93600 Aulnay- Sous- Bois (FR)

(74) Mandataire: Tonnellier, Marie- José, ROUSSEL- UCLAF 111, route de Noisy Boîte Postale no.9, F-93230 Romainville (FR)

EP 0 107 570 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1986

**Description**

L'invention concerne de nouveaux dérives de l'acide cyclopropane carboxylique susbtitué en 3 par une chaîne vinylique elle-même substituée, leur procédé et leurs intermédiaires de préparation, leur application comme pesticides et les compositions les renfermant.

L'invention a ainsi pour objet:

sous toutes leurs formes stéréoisomères ou sous forme de mélanges de stéréoisomères, les composés de formule générale (I):

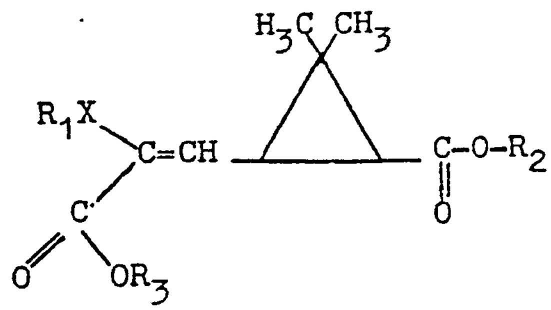

(I)

formule dans laquelle X représente un atome d'oxygène ou de soufre, un groupement sulfoxyde, et

ou bien $R_1$ et $R_3$, identiques ou différents, représentent des radicaux alkyles linéaires, ramifiés ou cyclisés, saturés ou insaturés, dont la chaîne peut être interrompue par un ou plusieurs hétéro-atomes, ou substituée par un ou plusieurs atomes d'halogène,

ou bien $R_1$ et $R_3$, identiques ou différents, réprésentent un radical aryle ou aralkyle renfermant de 6 à 18 atomes de carbone et $R_2$ représente le reste d'un alcool $R_2$-OH utilisé dans la synthèse des pyréthrinoïdes et notamment,

les composés de formule I dans lesquels X représente un atome d'oxygène ou de soufre.

L'invention a tout particulièrement pour objet les composés de formule I dans lesquels $R_2$ représente l'un des radicaux suivants:

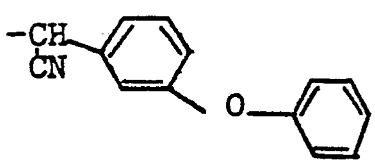
,
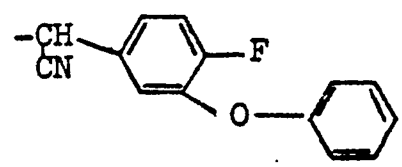

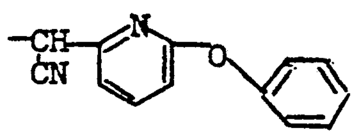
,
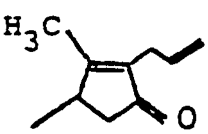

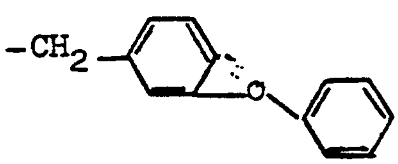

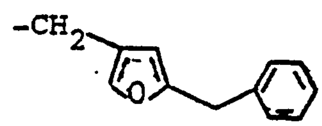

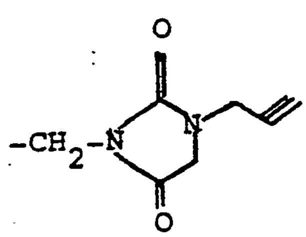
,
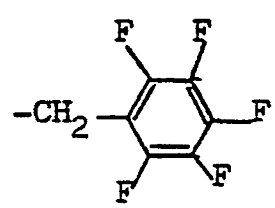

Dans les composés de formule (I), $R_1$ et $R_3$ peuvent représenter notamment des radicaux méthyle, éthyle, propyle linéaire ou ramifié, butyle linéaire ou ramifié, pentyle linéaire ou ramifié, hexyle linéaire ou ramifié, cyclopropyle, cyclobutyle, cyclopentyle, cyclohexyle, 1-propényle, 1-buténylе, 1, 3-butadiényle, 1-penténylе, 1-cyclobuténylе, 1-cyclopenténylе, 1-cyclohexénylе, un groupement $-CH_2-O-CH_3$, un groupement $-CH_2-CH_2-O-CH_3$, un groupement $-CH_2-NH-CH_3$, un groupement $-CH_2-CH_2-NH-CH_3$, un groupement $-CH_2-S-CH_3$, un groupement $CH_2-CH_2-S-CH_3$,

un groupement 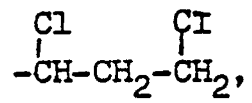

un groupement 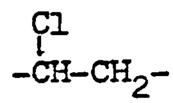

un groupement 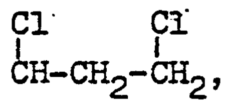

un groupement 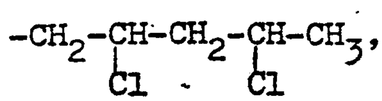

un groupement 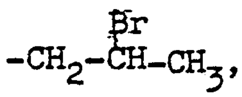

un groupement 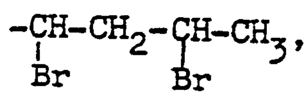

un groupement 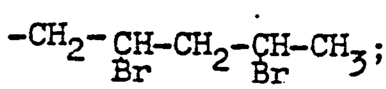

$R_1$ et $R_3$ peuvent aussi représenter notamment un radical phényle, tolyle, xolyle, benzyle, un groupement

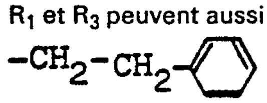

un groupement 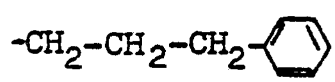

L'invention a tout spécialement pour objet les produits de formule I dont la préparation est donnée plus loin dans la partie expérimentale et notamment les produits dont les noms suivent:

le 1R trans 3( ΔZ 2-méthoxy 2-méthoxycarbonyl éthényl) 2,2-diméthyl cyclopropane carboxylate de (S)α-cyano 1-(3 -phénoxy phényl) méthyle;

le 1R trans 3( ΔZ 2-éthoxy 2-éthoxycarbonyl éthényl) 2,2-diméthyl cyclopropane carboxylate de (S)α-cyano 1(3 -phénoxy phényl) méthyle;

le 1R trans 3( ΔZ 2-méthoxy 2-méthoxcy carbonyl éthényl)2,2-diméthyl cyclopropane carboxylate de 3'allyl 2'méthyl 4'oxo 2'cyclopenten - 1'yle;

le 1R trans 3( ΔE 2-méthoxy 2-méthoxycarbonyl éthényl) 2,2-diméthyl cyclpropane carboxylate de 3'allyl 2'méthyl 4' oxo 2'cyclopenten - 1'yle;

le 1R trans 3( ΔZ 2-méthoxy 2-méthoxy carbonyl éthényl) 2,2-diméthyl cyclopropane carboxylate de /1(3-propyn-2-yl) 2,5 dioxo imidazolidinyl/méthyle;

le 1R trans 3/( ΔZ ou ΔE) 2-méthoxy 2-méthoxy carbonyl éthényl/ 2,2-diméthyl cyclopropane carboxylate de α(S) cyano 1(3-phénoxy 4-fluorophényl) méthyle;

le 1R trans 3/( ΔZou ΔE) 2-méthoxy 2-méthoxy éthényl/ 2,2-diméthyl cyclopropane carboxylate de α(RS) cyano 6'phénoxy 2'pyridylméthyle;

le 1R trans 3( ΔZ 2-méthoxy 2-éthoxycarbonyl éthényl) 2,2-diméthyl cyclopropane carboxylate d'α (S) cyano 1(3-phenoxy phényl) méthyle;

le 1R, trans 3( ΔZ 2-méthoxy 2-méthoxycarbonyl éthényl) 2,2-diméthyl cyclopropane carboxylate d'α(S)cyano 1(3-phénoxyphényl) méthyle;

le 1R,cis 3-( ΔZ 2-méthylsulfinyl 2-méthoxycarbonyléthényl) 2,2-diméthyl cyclopropane carboxylate d'α(S) cyano 1-(3-phénoxy phényl) méthyle (stéréoisomères A et B);

le 1R,trans 3-( ΔE 2-tertbutoxy 2-méthoxycarbonyléthényl) 2,2-diméthyl cyclopropane carboxylate d'α (S)

cyano 1-(3-phénoxy phényl) méthyle.

L'invention a aussi pour objet un procédé de préparation des composés de formule (1) caractérisé en ce que l'on fait réagir

un composé de formule (II): 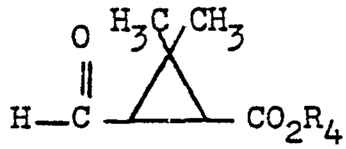 ( II )

dans laquelle $R_4$ représente un atome d'hydrogène ou le reste d'un alcool facilement clivable, pouvant se présenter dans le cas où $R_4$ est un atome d'hydrogène et la configuration est cis, sous la forme cyclisée:

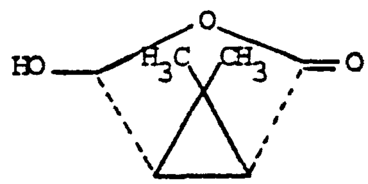

avec un composé de formule (III):

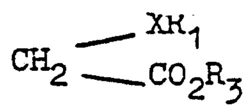 ( III )

dans laquelle $X, R_1$ et $R_3$ sont définis comme précédemment pour obtenir un composé de formule (IV):

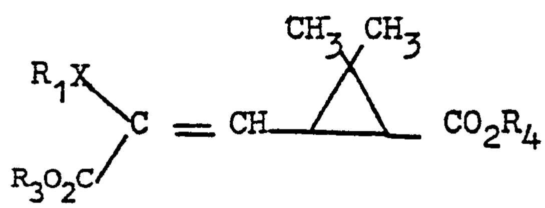 (IV )

que l'on soumet, dans le cas où $R_4$ représente un reste d'ester facilement clivable, à l'action d'un agent d'hydrolyse pour obtenir le composé de formule (IV) dans lequel $R_4$ représente un atome d'hydrogène, puis soumet ce composé de formule (IV) dans laquelle $R_4$ représente un atome d'hydrogène à l'action d'un alcool $R_2OH$ ou de l'un de ses dérivés pour obtenir le composé de formule (I) correspondant.

Dans des conditions préférentielles d'exécution du procédé ci-dessus:

- la réaction avec le produit de formule (III) est effectuée en présence d'une base forte telle qu'un alcoolate alcalin comme le tertbutylate de potassium ou l'éthylate de sodium;
- le reste d'alcool facilement clivable est un reste méthoxyméthyle, méthylthiométhyle ou tertbutyle;
- l'agent d'hydrolyse que l'on fait réagir sur l'ester facilement clivable est notamment un agent d'acidolyse tel que l'acide paratoluène sulfonique, l'acide trifluoroacétique ou l'acide chlorhydrique;
- la condensation de l'alcool $R_2OH$ et du composé de formule (IV) dans lequel $R_4$ représente un atome d'hydrogène est avantageusement effectuée en présence de dicyclohexylcarbodiimide ou de diisopropylcarbodiimide.

L'invention a également pour objet une variante du procédé precédent, caractérisée en ce que pour la préparation du composé de formule (IV) l'on soumet le composé de formule (II) à l'action d'un composé de formule (V):

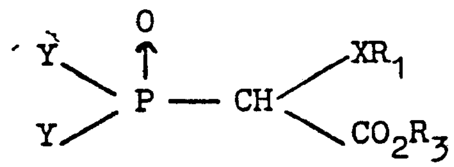 (V)

dans laquelle X, $R_1$ et $R_3$ conservent la même signification que précédemment et Y représente un radical alcoxyle ou aryle renformant jusqu'à 8 atomes de carbone.

Dans un mode de réalisation préféré, on fait réagir le composé de formule (II) avec le composé de formule (V) en presence d'une base forte comme le butyl lithium.

L'invention a également pour objet une variante du procédé tel que défini ci-dessus, pour la préparation des composés de formule (I) dans laquelle X représente un groupement sulfoxyde, caractérisée en ce que l'on utilise au départ un composé de formule (III) ou (V) dans laquelle X représente un atome de soufre et que l'on effectue l'oxydation au niveau du composé de formule (I) ainsi obtenu, dans lequel X représente un atome de soufre.

Dans des conditions préférentielles d'exécution du procédé ci-dessus, l'agent d'oxydation est le métapériodate de sodium ou un peracide tel que l'acide perbenzoïque ou métachloroperbenzoïque.

Les composés de formule (II), (III) et (V) sont décrits dans la littérature.

Les composés de formule (IV) et notamment de formule (IV'):

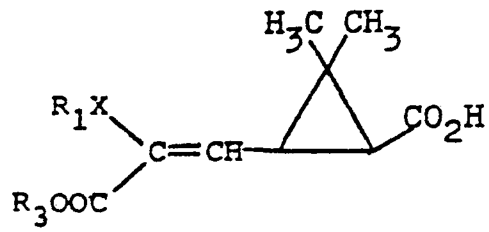 (IV')

dans laquelle $R_1$, $R_3$ et X sont définis comme précédemment sont des produits chimiques nouveaux, l'invention a donc pour objet ces produits à titre de produits chimiques nouveaux.

L'invention a notamment pour objet les produits de formule (IV') à titre de produits intermédiaires nécessaires à la mise en oeuvre du procédé de l'invention.

L'invention a plus particulièrement pour objet à titre de produits intermédiaires nécessaires à la mise en oeuvre du procédé de l'invention les acides de formule (IV) dont la préparation est donnée dans la partie expérimentale et tout particulièrement ceux dont les noms suivent:

- l'acide 1R trans 3-( $\Delta$ E 2-méthoxy 2-méthoxycarbonyl éthényl) 2,2-diméthyl cyclopropane carboxylique et l'acide 1R, trans 3-( $\Delta$Z 2-méthoxy 2-méthoxycarbonyl éthényl) 2,2-diméthyl cyclopropane carboxylique;
- l'acide 1R trans 3-( $\Delta$Z 2-méthylthio 2-éthoxycarbonyl éthényl) 2,2-diméthyl cyclopropane carboxylique;
- l'acide 1R trans 3-( $\Delta$E ou $\Delta$Z 2-phénylthio 2-éthoxycarbonyl éthényl) 2,2-diméthyl cyclopropane carboxylique;
- l'acide 1R trans 3-( $\Delta$E 2-éthoxy 2-éthoxycarbonyl éthényl) 2,2-diméthyl cyclopropane carboxylique et l'acide 1R trans 3-( $\Delta$Z 2-éthoxy 2-éthoxy carbonyl éthényl) 2,2-diméthyl cyclopropane carboxylique;
- l'acide 1R, cis 3-( $\Delta$Z 2-phényl thio 2-éthoxy carbonyl éthényl) 2,2-diméthyl cyclopropane carboxylique;
- l'acide 1R, cis 3-( $\Delta$E 2-méthoxy carbonyl 2-méthoxy éthényl) 2,2-diméthyl cyclopropane carboxylique et l'acide 1R, cis $\Delta$Z correspondant;
- l'acide 1R,cis 3-( $\Delta$Z 2-méthylthio 2-méthoxycarbonyléthényl) 2,2-diméthyl cyclopropame carboxylique et l'acide $\Delta$E correspondant;
- l'acide 1R,trans 3-( $\Delta$E 2-méthoxy 2-tertbutoxycarbonyléthenyl) 2,2-diméthyl cyclopropane carboxylique et l'acide $\Delta$Z correspondant.

Les composés de formule (I) présentent d'intéressantes propriétés qui permettent leur utilisation dans la lutte contre les parasites. Il peut s'agir par exemple de la lutte contre les parasites des végétaux, les parasites des locaux et les parasites des animaux à sang chaud. C'est ainsi que l'on peut utiliser les produits de l'invention pour lutter contre les insectes, les nématodes et les acariens parasites des végétaux et des animaux.

L'invention a pour objet l'application des composés de formule (I) à la lutte contre les parasites des végétaux, les parasites des locaux et les parasites des animaux à sang chaud.

L'invention a donc également pour objet les compositions destinées à la lutte contre les parasites des végétaux, les parasites des locaux et les parasites des animaux à sang chaud, caractérisées en ce qu'elles renferment comme principe actif au moins un des produits définis précédemnent.

Les produits de formule (I) peuvent être utilisés notamment pour lutter contre les insectes dans le domaine agricole, pour lutter par exemple contre les pucerons, les larves de lépidoptères et les coléoptères. Ils sont utilisés à des doses comprises entre 10 g et 300 g de matière active à l'hectare.

Les produits de formule (I) peuvent également être utilisés pour lutter contre les insectes dans les locaux, pour lutter notamment contre les mouches, les moustiques et les blattes.

L'invention a donc notamment pour objet les compositions insecticides renfermant comme principe actif au moins l'un des produits définis précédemment.

Dans les compositions insecticides selon l'invention, la ou les matières actives peuvent être additionnées éventuellement d'un ou plusieurs autres agents pesticides. Ces compositions peuvent se présenter sous forme de poudres, granules, suspensions, émulsions, solutions, solutions pour aérosols, bandes combustibles, appâts ou autres préparations employés classiquement pour l'utilisation de ce genre de composés.

Outre le principe actif, ces compositions contiennent, en général, un véhicule et/ou un agent tensio-actif, non ionique, assurant, en outre, une dispersion uniforme des substances constitutives du mélange. Le véhicule utilisé peut être un liquide, tel que l'eau, l'alcool, les hydrocarbures ou autres solvants organiques, une huile minérale, animale ou végétale, une poudre, telle que le talc, les argiles, les silicates, le Kieselguhr ou un solide combustible, tel que la poudre de tabu (ou marc de pyrèthre).

Ainsi que mentionné précédemnent, les compositions selon l'invention peuvent contenir un agent synergisant des pyréthrinoïdes.

Pour exalter l'activité insecticide des composés selon l'invention, on peut ainsi les additionner des synergistes classiques utilisés en pareil cas, tel que le 1-(2, 5, 8-trioxa dodécyl 2-propyl 4,5-méthylènedioxy)benzène (ou butoxyde de pipéronyle), la N-(2-éthyl heptyl)bicyclo/2,2,1-/ 5-heptèn-2, 3-dicarboximide, le pipéronyl-bis-2-(2'-n-butoxy éthoxy) éthyl acétal (ou tropital).

Les compositions insecticides selon l'invention contiennent, de préférence, entre 0,005 % et 10 % en poids de matière active.

Selon un mode opératoire avantageux, pour un usage dans les locaux, les compositions insecticides selon l'invention sont utilisées sous forme de compositions fumigantes.

Les compositions insecticides selon l'invention peuvent alors être avantageusement constituées, pour la partie non active, d'un serpentin combustible, ou encore d'un substrat fibreux incombustible. Dans ce dernier cas, le fumigant obtenu après incorporation de la matière active est placé sur un appareil chauffant tel qu'un électroémanateur.

Dans le cas où l'on utilise un serpentin insecticide, le support inerte peut être, par exemple, composé de marc de pyrèthre, poudre de tabu (ou poudre de feuilles Machilus Thumbergil), poudre de tige de pyrèthre, poudre de feuille de cèdre, poudre de bois (telle que de la sciure de pin) amidon et poudre de coque de noix de coco.

La dose de matière active peut alors être, par exemple, de 0,03 à 1 % en poids.

Dans le cas où l'on utilise un support fibreux incombustible, la dose de matière active peut alors être, par exemple, de 0,03 à 95 % en poids.

Les compositions selon l'invention pour un usage dans les locaux peuvent aussi être obtenues en préparant une huile pulverisable à base de principe actif, cette huile imbibant la mèche d'une lampe et étant alors soumise à la combustion.

La concentration du principe actif incorporé à l'huile est, de préférence, de 0,03 à 95 % en poids.

Il a été trouvé par ailleurs que les produits de formule (I) possèdent d'intéressantes propriétes acaricides.

Des tests classiques sur le Tetranychus Urticae permettent de démontrer l'activité acaricide des composés de formule (I).

Ces tests montrent que les composés (I) possèdent une double action dans la lutte contre les acariens. Outre l'action létale classique ces composés sont doués d'une action répulsive particulièrement intéressantes sous l'angle écologique.

Les composés de formule (I) peuvent ainsi être utilisés pour préparer des compositions acaricides et l'invention a donc également pour objet les compositions acaricides renfermant comme principe actif au moins l'un des produits definis précédemment. Ces compositions pourront être notamment des compositions répulsives vis-à-vis des acariens parasites des végétaux.

Les compositions acaricides selon l'invention comme les compositions insecticides ci-dessus, peuvent éventuellement être additionnées d'un ou plusieurs autres agents pesticides et d'un agent synergisant.

Les compositions acaricides peuvent se présenter sous forme de poudre, granulés, suspensions, émulsions, solutions.

Pour l'usage acaricide, on utilise de préférence des poudres mouillables, pour pulvérisation foliaire, contenant de 1 à 80 % en poids de matière active ou des liquides pour pulverisation foliaire contenant de 1 à 500 g/l de matière active. On peut également employer des poudres pour poudrages foliaires, contenant de 0,05 à 10 % en poids de matière active.

Les composés de formule (I) se sont également avérés doués d'intéressantes propriétés nématicides.

Des tests sur Panagrellus Silusiae permettent de démontrer l'activité nématicide des composés de formule (I).

Les composés de formule (I) peuvent ainsi être utilisés pour préparer des compositions nématicides et l'invention a donc également pour objet les compositions nématicides renfermant comme principe actif au moins l'un des produits définis ci-dessus.

Pour l'usage nématicide, on utilise de préférence des liquides pour traitement des sols contenant de 300 à 500g/l de principe actif.

Les composés acaricides et nématicides selon l'invention sont utilisés, de préférence, à des doses comprises entre 1 et 100 g de matière active à l'hectare.

Les composés de formule (I) sont également doués de propriétés antifongiques.

Des tests sur Aerobacter Aerogenes, Pseudomonas Aeruginosa, Botrytis Cinerea, Fusarium Roseum permettent de démontrer l'activité antifongique du composé de formule (I).

Les composés de formule (I) peuvent ainsi être utilisés pour préparer des compositions antifongiques, renfermant, comme matière active, un au moins desdits composés.

Pour l'usage antifongique, on utilise de préférence des poudres pour pulvérisation foliaire, contenant de 25 à 95 % en poids de matière active ou des poudres pour poudrage foliaire, contenant de 2,5 à 99 % en poids de matière active.

Les compositions insecticides, acaricides, nématicides et antifongiques selon l'invention, sont préparées selon les procédés classiques de l'industrie agrochimique.

La société demanderesse a enfin trouvé que les produits de formule (I) possèdent des propriétés anti-acariennes qui permettent d'utiliser ces produits dans la lutte contre les acariens parasites des animaux et notamment dans la lutte contre les ixodidés et les sarcoptidés parasites des animaux.

L'activité acaricide des composés de formule (I) peut être démontrée par un test sur Rhipicephalus Sanguinens chez le chien.

Les composés de formule (I) peuvent être utilisés chez l'animal pour lutter notamment contre toutes sortes de gales, telles que la gale sarcoptique, la gale psoroptique et la gale chorioptique.

Les composes de formule (I) peuvent encore être utilisés pour lutter contre les poux.

Les composés de formule (I) permettent encore de lutter contre toutes sortes de tiques comme, par exemple, l'espèce Boophilus, l'espèce Hyalomnia, l'espèce Amblyoma et l'espèce Rhipicephalus.

L'invention a donc également pour objet les compositions acaricides renfermant comme principe actif l'un au moins des produits définis précédemment, caractérisées en ce qu'elles sont utilisées dans la lutte contre les parasites des animaux à sang chaud, notamment contre les tiques et les gales.

Ces compositions sont préparées selon les procédés classiques.

Ces compositions peuvent être appliquées selon les méthodes de bains, pulvérisations, ou selon la méthode dite méthode "pour on" qui consiste à appliquer une composition renfermant le produit actif, sur une petite partie seulement du corps de l'animal pour avoir un effet sur l'ensemble du corps de l'animal.

Lorsqu'il s'agit de lutter contre les acariens parasites des animaux, on peut incorporer les produits de l'invention dans des compositions alimentaires en association avec un mélange nutritif adapté à l'alimentation animale. Le mélange nutritif peut varier selon l'espèce animale. Il peut renfermer des céréales, des sucres, des grains, des tourteaux de soja, d'arachide et de tournesol, des farines d'origine animale, par exemple des farines de poissons, des acides aminés de synthèse, des sels minéraux, des vitamines et des antioxydants. L'invention a donc également pour objet les compositions destinées à l'alimentation animale renfermant comme principe actif au moins l'un des produits définis précédemment.

Les composés de formule (I) peuvent également être utilisés pour préparer des associations pesticides doués d'activité insecticide, acaricide ou nématicide et l'invention a pour objet les associations douées d'activité insecticide, acaricide ou nématicide, caractérisées en ce qu'elles contiennent comme matière active, d'une part un au moins des composés de formule générale (I) et d'autre part, un au moins des esters pyréthrinoïdes choisis dans le groupe constitué par les esters d'alléthrolones, d'alcool 3,4,5,6-tétrahydrophtalimido methylique, d'alcool 5-benzyl 3-furyl méthylique, d'alcool 3-phenoxy benzylique et d'alcools α-cyano 3-phénoxy benzyliques des acides chrysanthémiques, par les esters d'alcool 5-benzyl 3-furyl méthylique des acides 2,2-diméthyl 3-(2-oxo 3-tétrahydrothiophénylidène méthyl) cyclopropane-1-carboxyliques, par les esters d'alcool 3-phénoxy benzylique et d'alcools α-cyano 3-phénoxy benzyliques des acides 2,2-diméthyl 3-(2,2-dichloro-vinyl) cyclopropane-1-carboxyliques, par les esters d'alcools α-cyano 3-phénoxy benzyliques d'acides 2,2-diméthyl 3-(2,2-dibromovinyl) cyclopropane-1-carboxyliques, par les esters d'alcool 3-phénoxy benzylique des acides 2-parachlorophényl 2-isopropyl acétiques, par les esters d'alléthrones, d'alcool 3, 4,5,6-tétrahydrophtalimido méthylique, d'alcool 5-benzyl 3-furyl méthylique, d'alcool 3-phénoxy benzylique et d'alcools α-cyano 3-phénoxy benzyliques des acides 2,2-diméthyl 3-(1,2-2,2-tétrahaloéthyl) cyclopropane-1-carboxyliques dans lesquels "halo" représente un atome de fluor, de chlore ou de brome, étant entendu que les copules acides et alcools des esters pyréthrinoïdes ci-dessus peuvent exister sous toutes leurs formes stéréoisomères possibles.

Les associations selon l'invention présentent notamment l'intérêt soit de permettre par la polyvalence de leur action de combattre une gamme de parasites plus étendue, soit de manifester dans certains cas un effet de synergie.

L'ensemble des compositions de l'invention peut être additionné d'un des synergistes classiques des pesticides, notamment des synergistes auxquels il a été fait mention pour les compositions insecticides et l'invention a donc pour objet les compositions mentionnées ci-dessus, caractérisées en ce qu'elles renferment en outre un synergiste des pyréthrinoïdes.

Les exemples suivants illustrent l'invention sans toutefois la limiter.

**EXEMPLE 1: 1R, trans 3(ΔE 2-méthoxy 2-méthoxy carbonyl éthenyl) 2,2-diméthyl cyclopropane carboxylate d'α(S) cyano 1(3-phénoxy phényl) méthyle.**

**Stade A**

Acide 1R, trans 3(ΔE 2-méthoxy 2-méthoxy carbonyl éthényl) 2,2-diméthyl cyclopropane carboxylique(1R, trans, ΔZ $R_1=CH_3$ $R_2=H$ $R_3=CH_3$)

**Stade $A_1$**

1R, trans 3(ΔE et ΔZ 2-méthoxy 2-méthoxy carbonyl éthényl) 2,2-diméthyl cyclopropane carboxylate de terbutyle(1R, trans ΔE et ΔZ $R_1=CH_3$ $R_2=$terbutyle $R_3=CH_3$ $X=O$)

Dans une solution de 8,5 g de 0,0 diméthyl phosphonate de 1-méthoxy acétate de méthyle dans 80 $cm_3$ de tétrahydrofuran on introduit à -60° C, 21 $cm_3$ d'une solution de butyl lithium à 20 % dans le cyclohexane, agite pendant 10 minutes, on introduit à -60° C, 7,92 g de 1R, trans 3-formyl 2,2-diméthyl cyclopropane carboxylate de terbutyle en solution dans 25 $cm_3$ de tétrahydrofuran, agite pendant 5 heures à -60° C, verse le mélange réactionnel dans l'eau, agite, extrait à l'ether éthylique, sépare la phase organique par décantation, la concentre à sec par distillation sous pression réduite, chromatographie le résidu sur silice en éluant avec un mélange d'hexane et d'acétate d'éthyle 9/1 et obtient 1,23 g d'ester ΔZ et 6,77 g d'ester ΔE.

**Isomère ΔZ**

Spectre de R. M. N. (deutérochloroforme)
pics à 1,22-1,3 ppm attribués aux hydrogènes des méthyles geminés
pic à 1,47 ppm attribué à l'hydrogène des méthyles des terbutyles
pics à 1,57-1,67 ppm et 2,25 à 2,5 ppm attribués aux hydrogènes en position 1 et en position 3 du cyclopropyle
pics à 3,73 et 3,8 ppm attribués aux hydrogènes des méthoxyles
pics à 5,93-6,1 ppm attribués à l'hydrogène éthylénique

**Isomère ΔE**

Spectre de R. M. N. (deutérochloroforme)
pics à 1,13-1,28 ppm attribués aux hydrogènes des méthyles géminés
pics à 1,45 ppm attribués aux hydrogènes des méthyles du terbutyle
pics à 2,53-2,76 ppm attribués à l'hydrogène en position 3 du cyclopropyle
pics à 3,6-4,3 ppm attribués aux hydrogènes des méthyles des methoxyles
pics à 4,9-5,03 ppm attribués à l'hydrogène éthylénique.

**Stade $A_2$**

Acide 1R trans 3(ΔE 2-méthoxy 2-méthoxy carbonyl éthényl) 2,2-diméthyl cyclopropane carboxylique (1R, trans ΔE $R_1=CH_3$ $R_2=H$ $R_3=CH_3$ $X=O$)

Dans une solution de 2,13 g de 1R, trans 3( Δ E 2-méthoxy 2-méthoxy carbonyl éthényl) 2,2-diméthyl cyclopropane carboxylate de terbutyle dans 40 $cm_3$ de chlorure de méthylène, on introduit à 0°C, 9,1 $cm_3$ d'acide trifluoroacétique, agite pendant 3 heures à 0°C, introduit 90 $cm_3$ de cyclohexane, agite, concentre à sec par distillation sous pression réduite, chromatographie le résidu sur silice en éluant avec un mélange d'hexane et d'acétate d'éthyle (4/6) et obtient 0,87 g de produit attendu (F=78°C)

Spectre de R. M. N. (deutérochloroforme)
pics à 1,2-1,35 ppm attribués aux hydrogènes des méthyles geminés
pics à 1,44-1,53 ppm et 2,7-2,9 ppm attribués aux hydrogènes en 1 et 3 du cyclopropane
pics à 3,60-3,84 ppm attribués aux hydrogènes des méthoxyles
pics à 4,9-5,03 ppm attribués à l'hydrogène éthylénique.

**Stade A'$_2$**

Acide 1R trans 3(ΔZ 2-méthoxy 2-méthoxy carbonyl éthényl) 2,2-diméthyl cyclopropane carboxylique (1R, trans ΔZ $R_1 = CH_3$ $R_2 = H$ $R_3 = CH_3$ X = O)

Dans 190 $cm_3$ de toluène on introduit 19 g de 1R, trans 3 (ΔZ 2-méthoxy 2-méthoxy carbonyl éthényl) 2,2-diméthyl cyclopropane carboxylate de terbutyle, 1,9 g d'acide paratoluène sulfonique et 190 $cm_3$ de toluène, porte le mélange réactionnel au reflux, l'y maintient pendant 1 heure, concentre à sec sous pression réduite, ajoute de l'eau et de l'éther, agite, sépare par décantation la phase éthérée, concentre à sec par distillation sous pression réduite, chromatographie le résidu sur silice en éluant par un mélange d'hexane et d'acétate d'éthyle (4/6). On obtient 7 g de produit attendu.

Spectre de R. M. N. (deutérochloroforme)
pics à 1,26-1,37 ppm attribués aux hydrogènes des méthyles geminés
pics à 1,67-1,75 ppm attribués à l'hydrogène en position 1 du cyclopropyle
pics à 2,4-2,47-2,54-2,03 ppm attribués à l'hydrogène en position 3 du cyclopropyle
pics à 3,7-3,8 ppm attribués aux hydrogènes des méthoxyles
pics à 5,9-6,06 ppm attribués à l'hydrogène éthylénique
pics à 8,3 ppm attribué à l'hydrogène du carboxyle.

**Stade B**

1R, trans 3-(ΔE 2-méthoxy 2-méthoxy carbonyl éthényl) 2,2-diméthyl cyclopropane carboxylate d'α(S)cyano 1-(3-phénoxy phényl) méthyle

Dans 14 $cm^3$ de chlorure de méthylène on introduit 1,45 g de 1 chloro -N,N,2-triméthyl propényl amine, 1,7 g d'acide 1R, trans 3(ΔE 2-méthoxy 2-méthoxy carbonyl éthényl) 2,2-diméthyl cyclopropane carboxylique, agite pendant 30 minutes, introduit 1,7 g d'alcool α(S)cyano 1-(3-phénoxy phényl) méthylique et 1,47 g de pyridine en solution dans 14 $cm^3$ de chlorure de methylène, agite pendant 3 heures à 20° C, verse le mélange réactionnel dans une solution aqueuse N d'acide chlorhydrique, agite, sépare par décantation la phase organique, la lave à l'eau, la concentre à sec par distillation sous pression reduite, chromatographie le résidu sur silice en éluant avec un mélange hexane acétate d'éthyle (8/ 2) et obtient 2,7 g de produit recherché.

Spectre de R. M. N. (deutérochloroforme)
pics à 1,16-1,28 ppm attribués aux hydrogènes des méthyles geminés
pics à 1,5-1,6 ppm attribués à l'hydrogène en position 1 du cyclopropyle
pics à 2,74-2,83-2,88-2,97 ppm attribués à l'hydrogène en position 3 du cyclopropyle
pics à 3,6-3,85 ppm attribués aux hydrogènes des methoxyles
pics à 4,9-5,0 ppm attribués à l'hydrogène éthylenique
pic à 6,45 ppm attribué à l'hydrogène porté par le carbone en α du CN.

**EXEMPLE 2: 1R, trans (ΔE 2-méthoxy 2-méthoxy-carbonyl-éthenyl) 2,2-diméthyl cyclopropane carboxylate de 1(3-phénoxy phényl) méthyle.**

On mélange 2,28 g d'acide 1R trans (ΔE 2-méthoxy 2-méthoxy carbonyl éthényl) 2,2-diméthyl cyclopropane carboxylique, 20 $cm^3$ de chlorure de méthylène, 2,06 g de dicyclohexylcarbodiimide, 1 $cm^3$ de pyridrine, agite pendant 45 minutes à 20°C introduit une solution de 2 g d'alcool 3-phénoxy benzylique dans 5 $cm^3$ de chlorure de méthylène, agite pendant 16 heures à 20°C, filtre, concentre le filtrat à sec par distillation sous pression réduite, chromatographie le résidu sur silice en éluant avec un mélange d'hexane et d'acétate d'éthyle (7/3) et obtient 1,6 g de produit attendu $/\alpha/_D = +3,5°$ (c = 1 %, chloroforme).

Spectre de R. M. N. (deutérochloroforme)
pics à 1,16-1,3 ppm attribués aux hydrogènes des méthyles géminés
pics à 1,48-1,57 ppm attribués à l'hydrogène en position 1 du cyclopropyle
pics à 2,67-2,76 ppm et 2,8-2,89 ppm attribués à l'hydrogèneen position 3 du cyclopropyle
pics à 3,58-3,8 ppm attribués aux hydrogènes des méthoxyles
pics à 4,9-5,03 ppm attribués à l'hydrogène éthylénique
pic à 5,1 ppm attribué à l'hydrogène du $CH_2$ benzylique
pics de 6,8 à 7,6 attribués à l'hydrogène des noyaux aromatiques.

En opérant comne à l'exemple 2 et en utilisant les alcools appropriés, on a obtenu les produits suivants (Exemples 3 à 14).

**EXEMPLE 3: 1R trans 3(ΔZ 2-méthoxy 2-méthoxy carbonyl éthényl) 2,2-diméthyl cyclopropane carboxylate de (S)α-cyano 1(3-phénoxy phényl) méthyle.**

(1R, trans ΔZ $R_1=CH_3$ $R_2=$

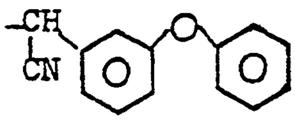

$R_3=CH_3$ X=O)
$(\alpha)_D=+7°5$(C=0,8 % $CHCl_3$)
Spectre de R. M. N. (deutérocholoroforme)
pics à 1,22-1,27 ppm attribués aux hydrogènes des méthyles géminés
pics à 1,7-1,8 ppm et de 2,4 à 2,7 ppm attribués aux hydrogènes en position 1 et en position 3 du cyclopropyle
pics à 3,7-3,8 ppm attribués aux hydrogènes des méthoxyles
pics à 5,9-6,0 ppm attribués à l'hydrogène éthylénique
pic à 6,4 ppm attribué à l'hydrogène porté par le carbone en α du CN
pics à 7,0-7,6 ppm attribués aux hydrogènes de noyaux aromatiques.

**EXEMPLE 4: 1R, trans (ΔZ 2-methoxy 2-méthoxy carbonyl éthenyl) 2,2-diméthyl cyclopropane carboxylate de 1(3-phénoxy phényl) méthylé.**

(1R, trans ΔZ $R_1=CH_3$ $R_2=$

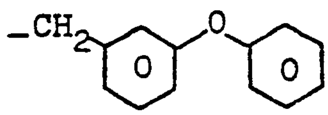

$R_3=CH_3$ X=O)
$(\alpha)_D=+10°$ (c= 0,5 %,chloroforme)
Spectre de R. M. N. (deutérochloroforme)
pics à 1,22-1,3 ppm attribués aux hydrogènes des méthyles géminés
pics à 1,6-1,7 ppm attribués à l'hydrogène en position 1 du cyclopropyle
pics à 2,36-2,6 ppm attribués à l'hydrogène en position 3 du cyclopropyle
pics à 3,7-3,8 ppm attribués aux hydrogènes des méthoxyles
pic à 5,1 ppm attribué aux hydrogènes du méthylène benzylique
pics à 5,9-6,1 ppm attribués à l'hydrogène éthylénique
pics à 6,9-7,6 ppm attribués aux hydrogènes des noyaux aromatiques.

**EXEMPLE 5: 1R, trans(ΔE 2-méthoxy 2-méthoxy carbonyl éthényl) 2,2-diméthyl cyclopropane carboxylate de 3'allyl 2'-méthyl 4' oxo 2'cyclopenten 1'yle**

(1R,trans ΔE $R_1=CH_3$ $R_2=$

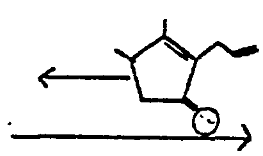

$R_3=CH_3$ X=O)
(F=62° C), $(\alpha)_D=-9,5°$ (c=0,5 %, chloroforme)
Spectre de R. M. N. (deutérochloroforme)
pics à 1,18–1,32 ppm attribués aux hydrogènes des méthyles géminés
pics à 1,45-1,54 ppm attribués à l'hydrogène en position 1 du cyclopropyle
pic à 2,06 ppm attribué au méthyle en 2' de l'alléthrolone

pics à 2,7-3,0 ppm attribués à l'hydrogène en position 3 du cyclopropyle
pics à 3,6-3,8 ppm attribués aux hydrogènes des méthoxyles.

**EXEMPLE 6:1R, trans ($\Delta$Z 2-méthoxy 2-méthoxy carbonyl éthényl) 2,2-diméthyl cyclopropane carboxylate de 3'allyl 2'méthyl 4' oxo 2'cyclopenten 1'yle**

(1R, trans $\Delta Z$ $R_1 = CH_3$ $R_2 =$

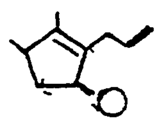

$R_3 = CH_3$ $X = O$)
$(\alpha)_D = -5,5°$ (c=0,5%, chloroforme)
Spectre de R. M. N. (deutérochloroforme)
pics à 1,25-1,32 ppm attribués aux hydrogènes des méthyles géminés
pics à 1,68-1,77 ppm attribués à l'hydrogène en position 1 du cyclopropyle
pic à 2,05 ppm attribués aux hydrogènes du méthyle en 2' de l'allethrolone
pics à 3,74-3,82 ppm attribués aux hydrogènes des méthoxyles
pics à 4,8-5,25 ppm attribués aux hydrogènes du méthylène en position 3 de l'allyle
pics à 5,5-6,2 ppm attribués à l'hydrogène en position 2' de l'alléthrolone
pics à 5,5-6,2 ppm attribués à l'hydrogène en position l'de l'alléthrolone
pics à 5,9-6,1 ppm attribués à l'hydrogène éthylénique.

**EXEMPLE 7: 1R, trans ($\Delta$Z 2-méthoxy 2-méthoxy carbonyl éthenyl) 2,2-diméthyl cyclopropane carboxylate de /1(3-propyn-2-yl) 2,5 dioxo imidazolidinyl méthyle/**

(1R trans, $\Delta Z$ $R_1 = CH_3$ $R_2 =$

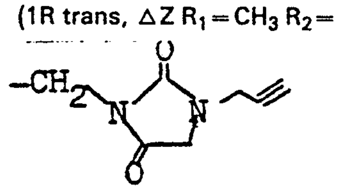

$R_3 = CH_3$ $X = O$)
$(\alpha)_D = +3,5°$ (c=0,5 %, chloroforme)
Spectre de R. M. N. (deutérochloroforme)
pics à 1,2-1,3 ppm attribués aux hydrogènes des méthyles géminés
pics à 1,6-1,7 ppm attribués à l'hydrogène en position 1 du cyclopropyle
pics à 2,3-2,4-2,45 ppm attribués à l'hydrogène de l'éthynyle
pics à 2,35-2,45-2,5-2,6 ppm attribués à l'hydrogène en position 3 du cyclopropyle
pics à 3,7-3,8 ppm attribués aux hydrogènes des méthoxyles
pics à 4,05 ppm attribués aux hydrogènes du méthylène imidazolinique
pics à 4,25-4,30 ppm attribués aux hydrogènes du méthylène du propynyle
pics à 5,4-5,55-5,6-5,7 ppm attribués aux hydrogènes du méthylène de la copule alcoolique du

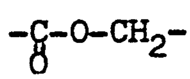

**EXEMPLE 8: 1R, trans ($\Delta$E 2-méthoxy 2-méthoxy carbonyl éthényl) 2,2-diméthyl cyclopropane carboxylate de (S) cyano 1(3-phénoxy 4-fluorophényl) méthyle. FIG24/20**

(1R, trans $\Delta E$ $R_1 = CH_3$ $R_2 =$ $R_3 = CH_3$ $X = O$)

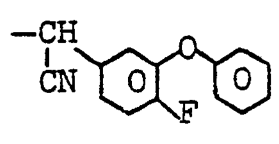

$(\alpha)_D = +3°$ (c=1,5 %, chloroforme)
Spectre de R. M. N. (deutérochloroforme)

pics à 1,16-1,25 ppm attribués aux hydrogènes des méthyles géminés
pics à 1,45-1,55 ppm attribués à l'hydrogène en position 1 du cyclopropyle
pics à 2,7–2,9 ppm attribués à l'hydrogène en position 3 du cyclopropyle
pics à 3,6-3,8 ppm attribués aux hydrogènes des méthoxyles
pic à 6,4 ppm attribués à l'hydrogène porté par le carbone en α du CN
pics à 6,9-7,6 ppm attribués aux hydrogènes des noyaux aromatiques.

**EXEMPLE 9: 1R, trans 3(ΔZ 2-méthoxy 2-méthoxy carbonyl éthényl) 2,2-diméthyl cyclopropane carboxylate de (S) cyano 1(3-phénoxy 4-fluorophényl) méthyle.**

(1R trans, ΔZ $R_1=CH_3$ $R_2=$ $R_3=CH_3$ X=O)

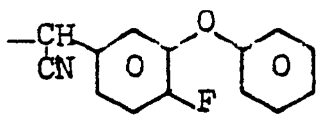

$(\alpha)_D=$ +9,5° (c=1,2 %, chloroforme)
Spectre de R.M.N. (deutérochloroforme)
pics à 1,22-1,25 ppm attribués aux hydrogènes des méthyles géminés
pics à 1,69-1,78 ppm attribués à l'hydrogène en position 1 du cyclopropyle
pics à 2,4-2,66 ppm attribués à l'hydrogène en position 3 du cyclopropyle
pics à 2,75-3,82 ppm attribués aux hydrogènes des méthoxyles
pics à 5,9-6,06 ppm attribués à l'hydrogène éthylénique
pic, à 6,4 ppm attribués à l'hydrogène porté par le carbone en α du CN
pics à 7-7,6 ppm attribués à l'hydrogène des noyaux aromatiques.

**EXEMPLE 10: 1R, trans 3(ΔE 2-méthoxy 2-méthoxy carbonyl éthényl) 2,2-diméthyl cyclopropane carboxylate d'(RS) cyano 6'phénoxy 2'pyridyl méthyle.**

(1R trans, ΔE $R_1=CH_3$ $R_2=$ $R_3=CH_3$ X=O)

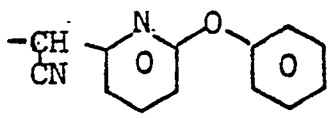

$(\alpha)_D=$ +6° (c=0,5 %, chloroforme)
Spectre de R. M. N. (deutérochloroforme)
pics à 1,18-1,2-1,28–1,35 ppm attribués aux hydrogènes des methyles géminés
pics à 1,52-1,60 ppm attribués à l'hydrogène en position 1 du cyclopropyle
pics à 2,68-3,0 ppm attribués à l'hydrogène en position 3 du cyclopropyle
pics à 3,6-3,78-3,8 ppm attribués aux hydrogènes des méthoxyles
pics à 4,9-5,0 ppm attribués à l'hydrogène éthylénique
pic à 6,4 ppm attribué à l'hydrogène porté par le carbone en α du CN
pics à 6,8-6,96-7-7,7 ppp attribues aux hydrogènes en 3 et 5 de la pyridine
pics à 7,65-7,76-7,88 ppm attribués à l'hydrogène en 4 de la pyridine.

**EXEMPLE 11: 1R, trans (ΔZ 2-méthoxy 2-méthoxy carbonyl éthenyl) 2,2-diméthyl cyclopropan e carboxylate d' (RS) cyano 6'phénoxy 2'pyridyl méthyle**

(1R, trans $\Delta Z$ $R_1 = CH_3$ $R_2 =$

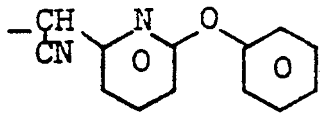

$R_3 = CH_3$ $X = O$)
$(\alpha)_D = +13°5$ (c = 0,7 %, chloroforme)
Spectre de R. M. N. (deutérochloroforme)
pics à 1,25-1,28-1,35 ppm attribués aux hydrogènes des méthyles géminés
pics à 1,73-1,82 ppm attribués à l'hydrogène en position 1 du cyclopropyle
pics à 2,4-2,67 ppm attribués à l'hydrogène en position 3 du cyclopropyle
pics à 3,57-3,77-3,8 ppm attribués aux hydrogènes des méthoxyles
pics à 5,9-6,08 ppm attribués à l'hydrogène éthylénique
pic à 6,38 ppm attribué à l'hydrogène porté par le carbone en α du CH
pics à 6,9-8 ppm attribués aux hydrogènes des noyaux aromatiques.

**EXEMPLE 12: 1R, trans 3($\Delta$Z 2-méthoxy 2-méthoxy carbonyl éthényl) 2,2-diméthyl cyclopropane carboxylate de benzyle.**

(1R trans, $\Delta Z$ $R_1 = CH_3$ $R_2 =$

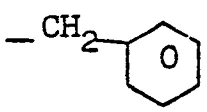

$R_3 = CH_3$ $X = O$)
Spectre de R. M. N. (deutérochloroforme)
pics à 1,22-1,3 ppm attribués aux hydrogènes des méthyles géminés
pics à 1,7-1,8 ppm attribués à l'hydrogène en position 1 du cyclopropyle
pics à 2,37-2,5 ppm attribués à l'hydrogène en position 3 du cyclopropyle
pics à 3,7-3,8 ppm attribués aux hydrogènes des méthoxyles
pic à 5,15 ppm attribué à l'hydrogène du methylène benzylique
pics à 5,9-6,06 ppm attribués à l'hydrogène éthylénique
pic à 7,38 ppm attribué aux hydrogènes des noyaux aromatiques.

**EXEMPLE 13: 1R, trans 3($\Delta$Z 2-méthoxy 2-méthoxy carbonyl éthenyl) 2,2-diméthyl cyclopropane carboxylate de penta fluoro phényl méthyle**

(1R, trans $\Delta Z$ $R_1 = CH_3$ $R_2 =$

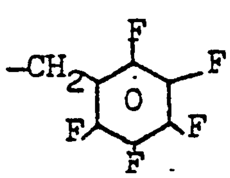

$R_3 = CH_3$ $X = O$)
Spectre de R. M. N. (deutérochloroforme)
pics à 1,22-1,32 ppm attribués aux hydrogènes des méthyles géminés
pics à 1,65-1,74 ppm attribués à l'hydrogène en position 1 du cyclopropyle
pics à 2,37-2,61 ppm attribués à l'hydrogène en position 3 du cyclopropyle
pics à 3,7-3,8 ppm attribués aux hydrogènes des méthoxyles
pics à 5,22-5,25-5,27 ppm attribués aux hydrogènes du méthylène benzylique
pics à 5,9-6,06 ppm attribués à l'hydrogène éthylénique.

**EXEMPLE 14: 1R, trans (△E 2-méthoxy 2-méthoxy carbonyl éthenyl) 2,2-diméthyl cyclopropane carboxylate de /1(3-propyn-2-yl) 2,5 dioxo imidazolidinyl/ méthyle**

(1R, trans △ $R_1 = CH_3$ $R_2 =$

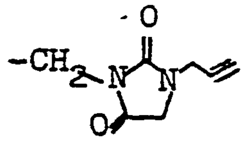

$R_3 = CH_3$ $X = O$)
$(\alpha)_D = 0$ (chloroforme)
Spectre de R. M. N. (deutérochloroforme)
pics à 1,15-1,32 ppm attribués aux hydrogènes des méthyles géminés
pics à 1,4-1,5 ppm attribués à l'hydrogène en position 1 du cyclopropyle
pics à 2,3-2,4-2,43 ppm attribues à l'hydrogène de l'éthynyl
pics à 2,65-2,87 ppm attribués à l'hydrogène en position 3 du cyclopropyle
pics à 3,6-3,8 ppm attribués aux hydrogènes des méthoxyles
pics à 4,28-4,32 ppm attribués aux hydrogènes du méthylène du propynyle
pics à 4,9-5,03 ppm attribués à l'hydrogène éthylénique
pics à 5,4-5,6-5,62-5,78 ppm attribues aux hydrogènes du éthylène du 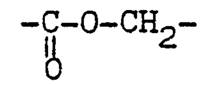

**EXEMPLE 15 et 15': 1R,cis3-(△Z et △E 2-methoxy 2-méthoxy carbonyl éthenyl-) 2,2-diméthyl cyclopropane carboxylate d' (S)cyano 1(3phénoxy phényl) méthyle.**

(1R, cis △Z et △E $R_1 = CH_3$ $R_2$

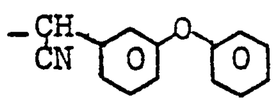

$R_3$-$CH_3$ X-0)

**Stade A**

Acide 1R, cis 3-(△E et △Z 2-méthoxy 2-méthoxy carbonyl éthényl) 2,2-diméthyl cyclopropane carboxylique (1R, cis △E et △Z $R_1 = CH_3$ $R_2 = H$ $R_3 = CH_3$ $X = O$)

On mélange 4,24 g de 0,0 diméthyl phosphonate de 1-méthoxy-acétate de méthyle, 2,84 g de 1R,5S 6,6-diméthyl 4(R) -hydroxy 3-oxo bicyclo /3.1.0/ héxane 2-one, 70 $cm_3$ de tétrahydrofuran, introduit à -45° C 4,5 g de terbutylate de potassium dans 30$cm_3$ de tétrahydrofuran, agite pendant 5 heures à -40° C, verse le mélange réactionnel dans l'eau glacée, acidifie à pH 5 pour une solution aqueuse N d'acide chlorhydrique, extrait à l'éther éthylique, concentre la solution éthérée à sec par distillatien sous pression réduite et obtient 4,2 g d'acide attendu.

Spectre de R. M. N. (deutérochloroforme)
pics à 1,29-1,33 ppm attribués aux hydrogènes des méthyles géminés
pics à 1,76-1,9 (E) et 1,8-1,96 (Z) ppm attribués à l'hydrogène en position 1 du cyclopropyle
pics à 2,2-2,5 (Z) et 2,75-3,05 (E) ppm attribués à l'hydrogène en position 3 du cyclopropyle
pics à 3,63-3,73 et 3,8 – 3,05 ppm attribués aux hydrogènes des méthoxyles
pics à 6,55-6,7 ppm (Z) et 5,58-5,73 ppm (E) attribués à l'hydrogène éthylénique.

**Stade B**

1R, cis 3-( ΔZ 2-méthoxy 2-méthoxy carbonyl éthényl) 2,2-dimétyl cyclopropane carboxylate d'(S)cyano 1-(3-phénoxy phényl) méthyle et 1R, cis 3( Δ E 2-méthoxy 2-méthoxy carbonyl éthényl) 2,2-diméthyl cyclopropane carboxylate d' (S)cyano 1(3-phénoxy phenyl) méthyle.

En operant comme à l'exemple 2, à partir de l'acide prepare au stade A et de l'alcool approprié et en separant les produits obtenus par chromatographie en éluant au mélange hexane-acétate d'éthyle 8/2, on obtient les 2 produits recherchés.

**Ester ΔE**

$(\alpha)_D = +7°$ (c= 0,3 %, chloroforme)
pics à 1,21-1,27 ppm attribués aux hydrogènes des méthyles géminés
pics à 1,83; 1,97 ppm attribués à l'hydrogène en position 1 du cyclopropyle
pics à 2,83-3,12 ppm attribués à l'hydrogène en position 3 du cyclopropyle
pics à 3,67-3,88 ppm attribués aux hydrogènes des méthoxyles
pics à 5,53-5,68 ppm attribués à l'hydrogène éthylénique
pic à 6,4 ppm attribué à l'hydrogène porté par le carbone en α du CN
pics à 7-7,6 ppm attribués aux hydrogènes des noyaux aromatiques

**Ester ΔZ:**

$(\alpha)_D$ +29'5° (c= 0,5 %, chloroforme)
Spectre de R. M. N. (deutérochloroforme)
pics à 1,26 ppm attribués aux hydrogènes des méthyles géminés
pics à 1,89–2,03 ppm attribués à l'hydrogène en position 1 du cyclopropyle
pics à 1,28-2,6 ppm attribués à l'hydrogène en position 3 du cyclopropyle
pics à 3,75-3,81 ppm attribués aux hydrogènes des méthoxyles
pic à 6,5 ppm attribué à l'hydrogène porte par le carbone en α du CN
pics à 6,5-6,7 ppm attribués à l'hydrogène éthylénique
pics à 6,98-7,7 ppm attribués aux hydrogènes des noyaux aromatiques.

**EXEMPLE 16:1Rtrans 3-( ΔZ-2-méthoxy 2-isopropyloxy carbonyl éthenyl) 2,2-diméthyl cyclopropane carboxylate d' (S) cyano 1(3 phénoxy phényl) méthyle.**

(1R, trans ΔZ $R_1 = CH_3$ $R_2 =$

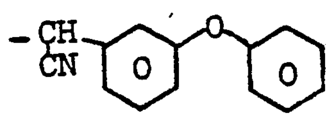

$R_3$=isopropyle X=O)

**Stade A**

Acide 1R trans 3( ΔZ 2-méthoxy 2-isopropyloxy carbonyl éthényl) 2,2-diméthyl cyclopropane carboxylique

**Stade $A_1$**

1R trans 3-( ΔZ 2-méthoxy 2-isopropyloxy carbonyl éthényl) 2,2-diméthyl cyclopropane carboxylate de méthoxy méthyle et le composé ΔE correspondant

(1R, trans $\Delta Z$ $R_1 = CH_3$ $R_2 = -CH_2OCH_3$ $R_3$-isopropyle X=O)

Dans 60 $cm^3$ de tétrahydrofuran on introduit 1,66 g d'oxyde de 1 isopropyloxy carbonyl 1-méthoxy méthyle diphényl phosphine, ajoute à -60°C, 2,75 $cm_3$ d'une solution de butyl lithium à 20% dans le cyclohexane, introduit une solution de 930 mg de 2,2-diméthyl 3-formyl cyclopropane carboxylate de méthoxy méthyle, dans 10 $cm^3$ de tétrahydrofuran, agite pendant 6 heures à -60°C, verse le mélange réactionnel dans l'eau, extrait à l'éther éthylique, concentre à sec par distillation sous pression réduite, chromatographie le résidu sur silice en éluant dans un mélange d'hexane et d'acétate d'éthyle (8/2) et obtient 650 mg d'isomère $\Delta Z$ et 380 mg d'isomère $\Delta E$

**Isomère $\Delta$Z:**

Spectre de R. M. N. (deutérochloroforme)
pics à 1,25-1,33 ppm attribués aux hydrogènes des méthyles géminés
pics à 1,31-1,35 ppm attribués aux hydrogènes des méthyles de l'isopropyle
pics à 1,7-1,80 ppm attribués à l'hydrogène en position 1 du cyclopropyle
pics à 2,57-2,63 ppm attribués à l'hydrogène en position 3 du cyclopropyle
pic à 3,5 ppm attribué aux hydrogènes du méthyl du méthoxy méthyle
pic à 3,7 ppm attribué aux hydrogènes du méthoxyle
pic à 5,08 ppm attribué à l'hydrogène de

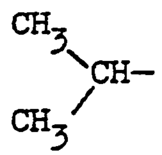

pic à 5,3 ppm attribué aux hydrogènes du méthylène du méthoxy méthyle

**Isomère $\Delta$E:**

Spectre de R. M. N. (deutérochloroforme)
pics à 1,22-1,35 ppm attribués aux hydrogènes des méthyles géminés
pics à 1,3-1,42 ppm attribués aux hydrogènes des méthyles de l'isopropyle
pics à 1,49-1,58 ppm attribués à l'hydrogène en position 1 du cyclopropyle
pics à 2,75-3 ppm attribués à l'hydrogène en position 3 du cyclopropyle
pic à 3,6 ppm attribué aux hydrogènes du méthoxyle
pic à 3,5 ppm attribué aux hydrogènes des méthyles du méthoxy méthyle
pics à 4,88-5,02 ppm attribués à l'hydrogène éthylénique
pic à 5,3 ppm attribués aux l'hydrogènes du méthylène du méthoxy méthyle.

**Stade $A_2$**

Acide 1R trans 3($\Delta Z$ 2-méthoxy 2-isopropyloxy carbonyl éthényl) 2,2-diméthyl cyclopropane carboxylique (1R, trans $\Delta Z$ $R_1 = CH_3$ $R_2 = H$ $R_3$=isopropyle X=O)

On mélange 5,4 g de 1R trans 3($\Delta Z$ 2-méthoxy 2-isopropyloxy carbonyl éthényl) 2,2-diméthyl cyclopropane carboxylate de méthoxy méthyle, 100 $cm^3$ de méthanol, 100 $cm^3$ d'acétone, 200 $cm^3$ de solution aqueuse N d'acide chlorhydrique, agite pendant 16 heures à 20°C introduit de l'eau, extrait au chlorure de methylène, concentre à sec par distillation sous pression réduite et obtient 4,6 g d'acide attendu.

Spectre de R. M. N. (deutérochloroforme)
pics à 1,27-1,38 ppm attribués aux hydrogènes des méthyles géminés
pics à 1,67-1,77 ppm attribués à l'hydrogène en position 1 du cyclopropyle
pics à 2,38-2,63 ppm attribués à l'hydrogène en position 3 du cyclopropyle
pic à 3,05 ppm attribué aux hydrogènes des méthyles de l'isopropyle
pic à 3,76 ppm attribué aux hydrogènes du méthoxyle
pic à 5,17 ppm attribué à l'hydrogène

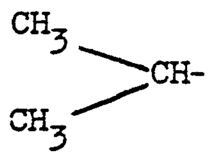

de l'isopropyle
pics à 5,9-6,08 ppm attribués à l'hydrogène éthylénique.

**Stade B**

1R, trans 3-(ΔZ 2–méthoxy 2-isopropyloxy carbonyl éthényl) 2,2-diméthyl cyclopropane carboxylate d' (S)cyano 1(3-phénoxy phényl) méthyle

En opérant comme à l'exemple 2, à partir de l'acide préparé au stade A et de l'alcool approprié, on obtient le produit recherché.

$(\alpha)_D = +9{,}5°$ (c=0,5 %, chloroforme)
Spectre de R. M. N. (deutérochloroforme)
pics à 1,22-1,27 ppm attribues aux hydrogènes des méthyles géminés
pics à 1,25-1,35 ppm attribués aux hydrogènes des méthyles de l'isopropyle
pics à 1,72-1,77 ppm attribués à l'hydrogène en position 1 du cyclopropyle
pics à 2,41-2,67 ppm attribués à l'hydrogène en position 3 du cyclopropyle
pic à 3,7 ppm attribué aux hydrogènes du méthoxyle
pic à 5,1 ppm attribué à l'hydrogène du

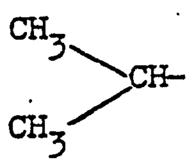

pics à 5,8 ppm et 5,96 ppm attribués à l'hydrogène éthylénique
pic à 6,4 ppm attribué à l'hydrogène porté par le carbone en α du CN
pics à 6,9-7,6 ppm attribués aux hydrogènes des noyaux aromatiques.

**EXEMPLE 17: 1R, trans 3(ΔZ 2-méthoxy 2-terbutoxy carbonyl éthenyl) 2,2-diméthyl cyclopropane carboxylate d' (S) cyano 1(3-phénoxy phényl) méthyle.**

(1R, trans ΔZ $R_1 = CH_3$ $R_2 =$

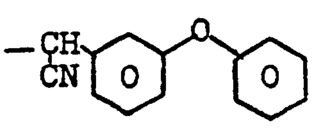

$R_3$ = terbutyle X = O)

**Stade A**

Acide 1R trans 3(ΔZ 2-méthoxy 2-terbutoxy carbonyl éthényl) 2,2-diméthyl cyclopropane carboxylique

**Stade $A_1$**

1R trans 3($\Delta$E 2-méthoxy 2-terbutoxy carbonyl éthényl) 2,2—di-méthyl cyclopropane carboxylate de méthoxy méthyle et ester $\Delta$Z correspondant

Dans 60 $cm^3$ de tétrahydrofurane on introduit 1,73g d'oxyde de 1-terbutoxy carbonyl 1-méthoxy méthyl diphényl phosphine, ajoute à -60°C, 2,75 $cm_3$ d'une solution de butyl lithium à 20 % dans le cyclohexane, introduit une solution de 930 mg de 2,2-diméthyl 3-formyl cyclopropane carboxylate de méthoxy méthyle dans 10 $cm_3$ de tétrahydrofurane,agite pendant 5 heures à -60°C, verse le mélange réactionnel dans l'eau, extrait par l'éther éthylique, concentre à sec par distillation sous pression réduite, chromatographie le résidu sur silice en éluant par in mélange hexane-acétate d'éthyle (8/2) et obtient 0,7 g d'ester $\Delta$Z et 0,72 g d'ester $\Delta$E.

**Ester $\Delta$Z**

Spectre de R. M. N. (deutérochloroforme)
pics à 1,25-1,35 ppm attribués aux hydrogènes des méthyles géminés
pics à 1,53 ppm attribué aux hydrogènes des méthyles du terbutyle
pics à 1,68-1,77 ppm attribués à l'hydrogène en position 1 du cyclopropyle
pics à 2,37-2,6 ppm attribués à l'hydrogène en position 3 du cyclopropyle
pic à 3,5 ppm attribué aux hydrogènes du méthyle du méthoxy méthyle
pic à 5,3 ppm attribué aux hydrogènes du méthylène du méthoxy méthyle
pics à 5,8-6 ppm attribués à l'hydrogène éthylénique.

**Ester $\Delta$E**

Spectre de R. M. N. (deutérochloroforme)
pics à 1,22-1,34 ppm attribués aux hydrogènes des méthyles gémines
pic à 1,56 ppm attribué aux hydrogènes des méthyles du terbutyle
pic à 3,5 ppm attribué aux hydrogènes du méthyle du méthoxy méthyle.

**Stade $A_2$**

Acide 1R, trans 3($\Delta$Z 2-terbutoxy carbonyl 2-méthoxy éthényl) 2,2—diméthyl cyclopropane carboxylique
(1R, trans $\Delta$Z $R_1=CH_3$ $R_2=H$ $R_3$=terbutyle X=O)

On mélange 520 mg de 1R, trans 3($\Delta$Z 2-méthoxy 2-terbutoxy carbonyl éthényl) 2,2-diméthyl cyclopropane carboxylate de méthoxy méthyle, 10 $cm^3$ de méthanol, 10 $cm^3$ d'acétone, 20 $cm^3$ de solution aqueuse N d'acide chlorhydrique, agite pendant 16 heures à 20°C, verse dans l'eau, extrait au chlorure de méthylène, concentre à sec par distillation sous pression réduite et obtient 420 mg d'acide attendu.

Spectre de R. M. N. (deutérochloroforme)
pics à 1,26—1,37 ppm attribués aux hydrogènes des méthyles géminés
pic à 1,55 ppm attribué aux hydrogènes des méthyles du ter butyle
pics à 1,65-1,74 ppm attribués à l'hydrogène en position 1 de l'isopropyle
pics à 2,37-2,6 ppm attribués à l'hydrogène en position 3 l'isopropyle
pic à 3,77 ppm attribué aux hydrogènes du méthoxyle
pics à 5,87-6,03 ppm attribués à l'hydrogène éthylénique.

**Stade B**

1R, trans 3-($\Delta$Z 2-méthoxy 2—terbutoxy carbonyl éthényl) 2,2-diméthyl cyclopropane carboxylate de (S) cyano 1-(3-phénoxy phényl) méthyle

En opérant comme à l'exemple 2, on obtient à partir de l'acide préparé au stade A, le produit recherché. (F=66°C), $(\alpha)_D$= +7;5 (c=0,5 %, chloroforme)

Spectre de R. M. H. (deutérochloroforme)
pic à 1,22 ppm attribué aux hydrogènes des méthyles géminés
pic à 1,53 ppm attribué aux hydrogènes des méthyles du terbutyle
pics à 1,7-1,79 ppm attribués à l'hydrogène en position 1 du cyclopropyle
pics à 2,42-2,66 ppm attribués à l'hydrogène en position 3 du cyclopropyle
pic à 3,73 ppm attribué, aux hydrogènes du méthoxyle

pics à 5,8-5,97 ppm attribués à l'hydrogène éthylénique
pic à 6,47 ppm attribué à l'hydrogène porté par le carbone en α du CH
pics à 6,9-7,58 ppm attribués aux hydrogènes des noyaux aromatiques.

**EXEMPLE 18: 1R trans 3(△Z 2-méthoxy 2-éthoxy carbonyl éthényl) 2,2-diméthyl cyclopropane carboxylate de (S)cyano 1(3-phénoxy phényl) méthyle.**

**Stade A**

Acide 1R, trans 3(△Z 2-méthoxy 2-éthoxy carbonyl éthényl) 2,2-diméthyl cyclopropane carboxylique:(1R trans (△Z) $R_1=CH_3$ $R_2=H$ $R_3=C_2H_5$ X=O)

**Stade $A_1$**

1R, trans 3(△Z 2-méthoxy 2-éthoxy carbonyl éthényl) 2,2-diméthyl cyclopropane carboxylate de terbutyle
(1R, trans △Z $R_1=CH_3$ $R_2$-terbutyle $R_3=C_2H_5$ X-O)
Dans 25 $cm_3$ de toluène on introduit 1,25 g de sodium coupe en petits morceaux, ajoute à 0°C, 0,05 $cm_3$ d'éthanol puis goutte à goutte 9,9 g de 2,2-diméthyl 3-formyl cyclopropane carboxylate de terbutyle et 17,7 g de méthoxy acétate d'éthyle, agite 16 heures à 20°C, verse le mélange réactionnel dans l'eau, extrait au chlorure de méthylène, concentre à sec par distillation sous pression réduite et chromatographie le résidu sur silice en éluant avec un mélange d'hexane et d'acétate d'éthyle et obtient 10 g de produit attendu.
Spectre de R. M. N. (deutérochloroforme)
pics à 1,2-1,3 ppm attribués aux hydrogènes des méthyles géminés
pic à 1,47 ppm attribué aux hydrogènes des méthyles du terbutyle
pics à 1,16-1,28-1,4 et 4,05-4,16-4,28-4,4 ppm attribués à l'hydrogène de l'éthyle de l'éthoxy
pics à 1,57-1,66 ppm attribués à l'hydrogène en position 1 du cyclopropyle
pics à 2,15-2,34 ppm et 2,4-2,5 ppm attribués à l'hydrogène en position 3 du cyclopropyle
pic à 3,7 ppm attribué aux hydrogènes du méthoxyle
pics à 5,86-6,03 ppm attribués à l'hydrogène éthylénique.

**Stade $A_2$**

Acide 1R, trans 3(△Z 2-méthoxy 2-éthoxy carbonyl éthényl) 2,2-diméthyl cyclopropane carboxylique
(1R, trans △Z $R_1$-$CH_3$ $R_2=H$ $R_3=C_2H_5$ X=O)
Dans 60 $cm_3$ de toluène on introduit 5,6 g de 1R, trans 3(△Z 2-méthoxy 2-éthoxy carbonyl éthényl) 2,2-diméthyl cyclopropane carboxylate de terbutyle, porte au reflux, ajoute 0,5 g d'acide para toluène sulfonique maintient pendant 1 heure au reflux, verse le mélange réactionnel dans l'eau, sépare par décantation la phase organique, extrait la phase aqueuse à l'éther éthylique, réunit les phases organiques, concentre à sec par distillation sous pression réduite, chromatographie le residu sur silice en éluant avec un mélange d'hexane et d'acétate d'éthyle (4/6) et obtient 3,2 g d'acide attendu.
Spectre de R. M. N. (deutérochloroforme)
pics à 1,25-1,36 ppm attribués aux hydrogènes des méthyles géminés
pics à 1,2-1,32-1,43 ppm et 4,03-4,15-4,26-4,38 ppm attribués aux hydrogènes de l'éthyle
pics à 1,65–1,74 ppm attribués à l'hydrogène en position 1 de l'isopropyle
pics à 2,35-2,44 et 2,52–2,61 ppm attribués à l'hydrogène en position 3 du cyclopropyle
pic à 3,7 attribué aux hydrogènes du méthyle du méthoxy
pics à 5,66-6,03 ppm attribués à l'hydrogène éthylénique
pic à 10,01 ppm attribué à l'hydrogène du carboxyle.

**Stade B**

1R, trans 3-(△Z 2–méthoxy 2-éthoxy carbonyl éthényl) 2,2-diméthyl cyclopropane carboxylate de (S) cyano 1-(3-phénoxyphényl) méthyle.
En opérant comme à l'exemple 2, on obtient le produit recherché à partir de l'acide obtenu au stade 13 et de l'alcool approprié.
Spectre de R.M.N. (deutérochloroforme)

pics à 1,21-1,26 ppm attribués aux hydrogènes des méthyles géminés
pics à 1,2-1,3-1,4 ppm et 4,1-4,2-4,3-4,4 ppm attribués aux hydrogènes de l'éthyle
pics a 1,7-1,8 et 2,43-2,5-2,6-2,7 ppm attribués aux hydrogènes en position 1 et 3 du cyclopropyle
pic à 3,74 ppm attribué aux hydrogènes du méthoxy
pics à 5,09-6,05 ppm attribués à l'hydrogène éthylénique
pic à 6,43 ppm attribué à l'hydrogène porté par le carbone en du CN.

**EXEMPLE 19: 1R trans 3 △Z (2-éthoxy 2-éthoxycarbonyléthényl) 2,2-diméthyl cyclopropane carboxylate de (S) cyano 1-)3-phénoxyphényl) méthyle.**

**Stade A**

Acide 1R trans 3-(△Z 2-éthoxy 2-éthoxycarbonyléthényl) 2,2-diméthyl cyclopropane carboxylique
(1R trans △Z $R_1=C_2H_5$ $R_2=H$ $R_3=C_2H_5$ $X=O$)

**Stade $A_1$**

1R,trans 3-(△E 2-éthoxy 2-éthoxycarbonyléthényl) 2,2-diméthyl cyclopropane carboxylate de terbutyle
(1R,trans △E $R_1$-$C_2H_5$ $R_2$=terbutyle $R_3=C_2H_5$ $X=O$ etl'isomère △Z)

Dans une solution de 5,36 g d'1-éthoxy 1-éthoxycarbonylméthyl phosphonate d'O,O diéthyle dans 40 $cm^3$ de tétrahydrofuranne, on introduit à -60°C, lentement, une solution de 10,5 $cm^3$ de butyl lithium dans le cyclohexane (titrant 2 moles/l), agite pendant 10 minutes, introduit à -60°C, 3,96 g de 1R,trans 3-formyl 2,2-diméthyl cyclopropane carboxylate de terbutyle, agite pendant 5 heures à -60°C, verse le mélange réactionnel dans l'eau, extrait à l'éther éthylique, concentre par distillation sous pression reduite, chromatographie le résidu sur silice en éluant dans un mélange d'hexane et d'acétate d'éthyle (9/1) et obtient 1,35 g de 1R,trans 3-(△Z 2-éthoxy 2-éthoxycarbonyléthényl) 2,2-diméthyl cyclopropane carboxylate de terbutyle et 3,46 g de 1R,trans 3-(△E 2-éthoxy 2-éthoxycarbonyléthényl) 2,2-diméthyl cyclopropane carboxylate de terbutyle.

**Isomère △Z:**

Spectre de R. M. N. (deutérochloroforme)
pics à 1,2-1,3 ppm attribués aux hydrogènes des méthyles géminés
pics à 1,18-1,43 ppm et 3,75-4,1 ppm attribués aux hydrogènes de l'éthoxyle
pics à 1,18-1,43 ppm et 4,06-4,43 ppm attribués aux hydrogènes de l'éthoxy carbonyle
pic à 1,47 ppm attribué aux hydrogènes des méthyles du terbutoxy
pics à 1,57-1,66 ppm attribués à l'hydrogène en 1 du cyclopropyle
pics à 2,27-2,36 ppm et 2,44-2,53 ppm attribués à l'hydrogène en position 3 du cyclopropyle
pics à 5,95-6,61 ppm attribués à l'hydrogène éthylénique.

**Isomère △E:**

Spectre de R. M. N. (deutérochloroforme)
pics à 1,06-1,28 ppm attribués aux hydrogènes des méthyles géminés
pics à 1,22-1,33-1,45 ppm et 3,6-3,7-3,8-3,9 ppm attribués aux hydrogènes de l'éthoxyle
pics à 1,22-1,33-1,45 ppm et 4,1-4,2-4,3–4,4 ppm attribués aux hydrogènes de l'éthoxy carbonyle
pics à 1,15-1,47 ppm attribués à l'hydrogène en position 1 du cyclopropyle
pic à 1,47 ppm attribué aux hydrogènes des méthyles du terbutoxy
pics à 2,53-2,62 ppm et 2,67–2,76 ppm attribués à l'hydrogène en position 3 du cyclopropyle
pics à 4,54-5,08 ppm attribués à l'hydrogène éthylénique.

**Stade $A_2$**

Acide 1R, trans 3(△Z 2-éthoxy 2-éthoxy carbonyl éthényl) 2,2-diméthyl cyclopropane carboxylique
1R, trans △Z $R_1=C_2H_5$ $R_2$-H $R_3=C_2H_5$ X-O)

Dans 32 $cm^3$ de toluène, on introduit 3,2 g de 1R, trans 3(△E 2-éthoxy 2-éthoxy carbonyl éthényl)2,2-diméthyl

cyclopropane carboxylate de terbutyle et 0,32 g d'acide para toluène sulfonique, porte le mélange réactionnel au reflux, l'y maintient pendant 45 minutes, concentre à sec par distillation sous pression réduite ajoute de l'éther éthylique lave à l'eau, concentre la solution organique à sec par distillation sous pression réduite, chromatographie le résidu sur silice en éluant dans un mélange d'hexane et d'acétate d'éthyle (4/6) et obtient 1,84 g d'acide attendu.

Spectre de R. M. N. (deutérochloroforme)
pics à 1,25-1,35 ppm attribués aux hydrogènes des méthyles géminés
pics à 1,2-1,3-1,4 ppm et 3,76-3,88-4,0-4,1 ppm attribués aux hydrogènes de l'éthoxyle
pics à 1,2-1,3-1,4 ppm et 4,08-4,2-4,3-4,4 ppm attribués aux hydrogènes de l'éthoxycarbonyle
pics à 1,65-1,75 ppm attribués à l'hydrogène en position 1 du cyclopropyle
pics à 2,4-2,49-2,56-2,65 ppm attribués à l'hydrogène en position 3 du cyclopropyle
pics à 5,95-6,11 ppm attribués à l'hydrogène éthylénique
pic à 7,33 ppm attribué à l'hydrogène du carboxyle.

**Stade B**

1R, trans 3-(ΔZ 2-éthoxy 2-éthoxy carbonyl éthényl) 2,2-dimétyl cyclopropane carboxylate de (S)cyano 1-(3-phénoxy phényl) méthyle

En opérant comme à l'exemple 2, à partir de l'acide préparé au stade A et de l'alcool approprié, on obtient le produit recherché.

Spectre de R. M. N. (deutérochloroforme)
pics à 1,2-1,95 ppm attribués aux hydrogènes des méthyles géminés
pics à 1,15-1,17-1,38 ppm et 3,75-3,87-3,98-4,1 ppm attribués aux hydrogènes de l'éthoxyle
pics à 1,15-1,17-1,38 ppm et 4,05-4,16-4,23-4,4 ppm attribués aux hydrogènes de l'éthoxy carbonyle
pics à 1,7-1,8 ppm attribués à l'hydrogène en position 1 du cyclopropyle
pics à 2,4-2,68 ppm attribués à l'hydrogène en position 3 du cyclopropyle
pics à 5,9-6,0 ppm attribués à l'hydrogène éthylénique
pics à 6,38 ppm attribués à l'hydrogène porté par le carbone en α du CN
pics à 6,9-7,5 ppm attribués aux hydrogènes des noyaux aromatiques.

**EXEMPLE 20: 1R trans 3(ΔE 2-éthoxy 2-éthoxy carbonyl éthenyl) 2,2-diméthyl cyclopropane carboxylate de (S) cyano 11,3-phénoxy phényl) méthyle.**

(1R trans ΔE $R_3$ et $R_1$ = éthyle $R_2$ =

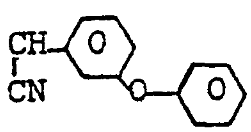

X = O)

**Stade A**

Acide 1R, trans 3(ΔE 2-éthoxy 2-éthoxy carbonyl éthényl) 2,2-diméthyl cyclopropane carboxylique (1R,trans ΔE $R_1 = C_2H_5$ $R_2 = H$ $R_3 = C_2H_5$ X = O)

Dans une solution de 3,38 g de 1R, trans 3(ΔE 2-éthoxy 2-éthoxy carbonyl éthényl) 2,2-dimétyl cyclopropane carboxylate de terbutyle dans 70 $cm^3$ de chlorure de méthylène, on introduit à 0°C 13,4 $cm^3$ d'acide trifluoroacétique,agite pendant 3 heures à 0°C, introduit du cyclohexane, concentre à sec par distillation sous pression réduite, chromatographie le résidu sur silice en éluant par un mélange d'hexane et d'acétate d'éthyle (4/6) et obtient 1,81 g d'acide attendu.

Spectre de R. M. N. (deutérochloroforme)
pics à 1,19-1,35 ppm attribués aux hydrogènes des méthyles geminés
pics à 1,23–1,35-1,46 ppm et 4,15-4,26-4,38-4,5 ppm attribués aux hydrogènes de l'éthoxy carbonyle
pics à 1,23–1,35-1,46 ppm et 3,6-3,7-3,8-3,95 ppm attribués aux hydrogènes de l'éthyloxyle
pics à 1,43-1,52 ppm attribués à l'hydrogène en position 1 du cyclopropyle
pics à 2,8-2,9-2,69-2,78 ppm attribués à l'hydrogène en position 3 du cyclopropyle

pics à 4,96-5,1 ppm attribués à l'hydrogène éthylénique
pic à 9 ppm attribué à l'hydrogène du carboxyle.

**Stade B**

1R trans 3-( △ E 2-éthoxy 2-éthoxy carbonyl éthényl) 2,2-diméthyl cyclopropane carboxylate de (S)cyano 1-(3-phénoxy phényl) méthyle.

En operant comme à l'exemple 1, à partir de l'acide prépare au stade A, on obtient le produit recherché.
$(\alpha)_D = +9{,}5°$ (c- 1 %, chloroforme)
Spectre de R. M. N. (deutérochloroforme)
pics à 1,15-1,25 ppm attribués aux hydrogènes des méthyles géminés
pics à 1,2-1,45 ppm et 4,15-4,26-4,38-4,5 ppm attribués aux hydrogènes de l'éthoxycarbonyle
pics à 1,2-1,45 ppm et 3,6-3,7-3,8-3,95 ppm attribués aux hydrogènes de l'éthoxyle
pics à 1,49-1,58 ppm attribués à l'hydrogène en position 1 du cyclopropyle
pics à 2,7-2,8-2,85-2,95 ppm attribués à l'hydrogène en position 3 du cyclopropyle
pics à 4,9-5,0 ppm attribués à l'hydrogène du noyau aromatique
pic à 6,43 ppm attribué à l'hydrogène porté par le carbone en α du CN
pics à 6,9-7,6 ppm attribués aux hydrogènes des noyaux aromatiques.

**EXEMPLE 21: 1R trans 3( △ E ou △ Z 2-phénoxy 2-éthoxy carbonyl éthényl) 2,2-diméthyl cyclopropane carboxylate de (S) cyano 1(3-phénoxy phényl) méthyle**

(1R trans △ E ou △ Z $R_1$ =

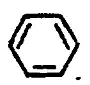

$R_2$ =

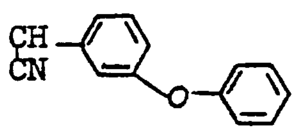

$R_3 = C_2H_5$ X = O)

**Stade A**

Acide (1R, trans) 3(2-phénoxy 2-éthoxy carbonyl éthényl) 2,2-diméthyl cyclopropane carboxylique

**Stade: $A_1$**

1R trans 3( △ E ou △ Z 2-phénoxy 2-éthoxy carbonyl éthényl) 2,2-diméthyl cyclopropane carboxylate de terbutyle
(1R, trans △ E ou △ Z $R_1$ =

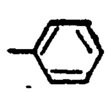

$R_2$ = terbutyle $R_3 = C_2H_5$ X = O)

Dans 25 $cm^3$ de toluène on introduit 1,25 g de sodium coupe en morceaux, introduit à 0° C 0,05 $cm^3$ d'éthanol puis goutte à goutte 9,9 g de 1R, trans 2,2-diméthyl 3-formyl cyclopropane carboxylate de terbutyle et 28 g de phénoxy acétate d'éthyle, agite pendant 16 heures à 20° C, ajoute 20 $cm^3$ de toluène, agite pendant 4 heures, verse le mélange réactionnel sur un mélange d'eau et de glace, extrait au chlorure de méthylène, concentre à sec par distillation sous pression réduite, chromatographie le résidu sur silice en eluant avec un mélange d'hexane et d'acétate d'éthyle (7/3) et obtient 12,47 g de produit attendu.

Spectre de R. M. N. (deutérochloroforme)
pics à 1,05-1,16-1,28 ppm et 3,96-4,08-4,2-4,3 ppm attribués aux hydrogènes de l'éthyle
pics à 1,15-1,23 ppm attribués aux hydrogènes des méthyles géminés
pic à 1,37 ppm attribué aux hydrogènes des méthyles du terbutyle
pics à 1,62-1,70 ppm attribués à l'hydrogène en position 1 du cyclopropyle
pics à 2,13-2,4 ppm attribués à l'hydrogène en position 3 du cyclopropyle
pics à 6,23-6,4 ppm attribués à l'hydrogène éthylénique
pics à 6,7-7,4 ppm attribués auxc hydrogènes du noyau aromatique.

**Stade $A_2$**

Acide (1R, trans) 3( △E ou △Z 2-phénoxy 2-éthoxy carbonyl éthenyl) 2,2-diméthyl cyclopropane carboxylique (1R, trans △Z ou △E R1 =

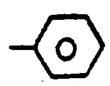

$R_2 = H$ $R_3 = C_2H_5$ $X = O$)

Dans 120 $cm_3$ de toluène on introduit 12 g de 1R, trans 3(2-phénoxy 2-éthoxy carbonyl éthényl) 2,2-diméthyl cyclopropane carboxylate de terbutyle, porte au reflux et ajoute 1 g d'acide para toluène sulfonique, maintient le reflux pendant 1 heure, verse le mélange réactionnel dans l'eau, sépare par décantation la phase organique, concentre à sec par distillation sous pression réduite, chromatographie le résidu sur silice en éluant avec un mélange d'hexane et d'acétate d'éthyle (4/6) et obtient 7,91 g d'acide attendu.

Spectre IR (chloroforme)
absorption à 3510 $cm^{-1}$ OH acide mono et dimère

absorption à 1720 $cm^{-1}$ 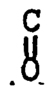 de l'ester

absorption à 1699 $cm^{-1}$ acide du dimère
absorption à 1655$cm^{-1}$ (épaulement)et 1651$cm^{-1}$(maximum) C=C conjugué
absorption à 1600 $cm^{-1}$, 1594 $cm^{-1}$, 1491 $cm^{-1}$ aromatique
absorption à 1380 $cm^{-1}$ gem. diméthyle.

**Stade B**

1R,trans 3( △E ou △Z 2-phénoxy 2-éthoxy carbonyl éthényl) 2,2-diméthyl cyclopropane carboxylate de (S)cyano 1(3-phénoxy phényl) méthyle

En opérant comme à l'exemple 2, à partir de l'acide préparé au stade A et de l'alcool approprié, on obtient le produit recherché.

Spectre de R. M. N. (deutérochloroforme)
pics à 1,06-1,18-1,3 ppm et 4,01-4, 13-4, 25-4,37 ppm attribués aux hydrogènes de l'éthyle
pics à 1,13-1,15 ppm attribués aux hydrogènes des méthyles géminés
pics à 1,8-1,9 ppm attribués à l'hydrogène en position 1 du cyclopropyle
pics à 2,3-2,4-2,46-2,55 ppm attribués à l'hydrogène en position 3 du cyclopropyle
pics à 6,27-6,46 ppm attribués à l'hydrogène éthylénique
pic à 6,36 ppm attribué à l'hydrogène porté par le carbone en α du CN.

**EXEMPLE 22: 1R, cis 3(△Z 2-méthyl thio 2-éthoxy carbonyl éthényl) 2,2-diméthyl cyclopropane carboxylate de (S) cyano 1(3phénoxy-phényl) méthyle.**

(1R, cis △Z $R_1=CH_3$ $R_2=$

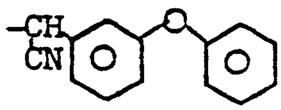

$R_3=C_2H_5$ X=S)

**Stade A**

Acide 1R, cis 3(△Z 2-méthyl thio 2-éthoxy carbonyl éthényl) 2,2-diméthyl cyclopropane carboxylique

**Stade $A_1$**

Acide 1R, cis 3-(1-hydroxy 2-méthyl thio 2-éthoxy carbonyl éthyl) 2,2-diméthyl cyclopropane carboxylique

Dans 200 $cm^3$ de tétrahydrofurane on mélange 11,54 g de méthyl thio acétate d'éthyle et 11,36 g de 1R, 5S 6,6-diméthyl 4(R)-hydroxy 3-oxa bicyclo /3.1.0/ hexane 2-one. On refroidit à -60°C pour introduire en 30 minutes 18 g de terbutylate de potassium dans 140 $cm_3$ de tétrahydrofurane. On agite 1 heure à -60°C et ajoute en 10 minutes sans depasser -20°C 200 $cm^3$ d'acide chlorhydrique N. On verse le mélange réactionnel dans 1,5 1 d'eau et extrait au chlorure de methylène. On concentre à sec sous pression réduite et recristallise le résidu dans l'ether isopropylique. On obtient 10,22 g de produit attendu (F=114°C).

**Stade $A_2$**

6,6-diméthyl 4-/méthyl thio, éthoxy carbonyl méthyl/ 3-oxa bicyclo /3.1.0./ hexane 2-one

On porte au reflux 9,67 g du produit obtenu au stade precédent dans 120 $cm_3$ de benzène en présence de 80 mg d'acide para toluène sulfonique. On distille environ 60 $cm_3$ de benzène, équipe le ballon d'un appareil de Dean Stark rempli de gel de silice et maintient le reflux 1 heure et 30 minutes. On concentre à sec sous pression réduite, chromatographie le résidu sur silice, élue avec un mélange d'hexane et d'acétate d'éthyle(6/4) et obtient 7,36 g de produit attendu.

**Stade $A_3$**

Acide 1R, cis 3-(△Z 2-méthyl thio 2-éthoxy carbonyl éthényl) 2,2 diméthyl cyclopropane carboxylique et acide 1R, cis 3-(△E 2-méthyl thio 2-méthoxy carbonyl éthényl) 2,2-diméthyl cyclopropane carboxylique

On porte au reflux 6,5 g du produit obtenu au stade $A_2$ dans 60 $cm^3$ de benzène avec 6 $cm^3$ de triéthylamine pendant 3 heures. On refroidit et verse le mélange réactionnel dans 100 $cm^3$ d'acide chlorhydrique 2N. On agite, décante la phase organique, concentre à sec, chromatographie le résidu sur silice, élue avec un mélange chlorure de méthylène acétate d'éthyle (8/2) et obtient 2,5 g d'acide △Z et 170 mg d'acide △E et 1,74g d'un mélange △E et △Z dont on récupère 220mg d'acide △E après une seconde chromatographie sur silice (éluant chlorure de méthylène-acétate d'éthyle: 9/1).

**Acide △E:**

Spectre de R. M. N. (deutérochloroforme)

pics à 1,2-1,3-1,4 ppm et 4,1-4,2-4,4-4,5 ppm attribués aux hydrogènes de l'éthyle

pics à 1,27-1,35 ppm attribués aux hydrogènes des méthyles géminés
pics à 1,8-1,9-2,8-2,9-3,1 ppm attribués aux hydrogènes en position 1 et 3 du cyclorpopyle
pic à 2,2 ppm attribué aux hydrogènes du thio méthyle
pics à 6,25-6,41 ppm attribués à l'hydrogène éthylénique.

**Acide △Z:**

Spectre de R. M. N. (deutérochloroforme)
pics à 1,2-1,3-1,4 ppm et 4,1-4,2-4,3-4,5 ppm attribués aux hydrogènes de l'éthyle
pics à 1,3-1,36 ppm attribués aux hydrogènes des méthyles géminés
pics à 1,9-2,05 et 2,5-2,6-2,8 ppm attribués aux hydrogènes en position 1 et 3 du cyclopropyle
pic à 2,3 ppm attribué aux hydrogènes du thiométhyle
pic à 6,5 ppm attribué à l'hydrogène du carboxyle
pics à 7,4-7,6 ppm attribués à l'hydrogène éthylénique.

**Stade B**

1R, cis 3(△Z 2-méthyl thio 2-éthoxy carbonyl ethenyl) 2,2-diméthyl cyclopropane carboxylate de (S)cyano 1-(3-phénoxcy phényl) méthyle

En opérant comme à l'exemple 2, à partir de l'acide preparé au stade A et de l'alcool approprié, on obtient le produit recherché.

Spectre de R. M. N. (deutérochloroforme)
pics à 1,22-1,33-1,45 ppm et 4,13-4,25-4,37-4,48 ppm attribués aux hydrogènes de l'éthyle
pics à 1,25-1,28 ppm attribués aux hydrogènes des méthyles géminés
pics à 1,97-2,10 ppm attribués à l'hydrogène en position 1 du cyclopropyle
pics à 2,55-2,65-2,74-2,85 ppm attribués à l'hydrogène en position 3 du cyclopropyle
pic à 6,46 ppm attribué à l'hydrogène porté par le carbone en α du CN
pics à 7,0-7,67 ppm attribués aux hydrogènes des noyaux aromatiques.

**EXEMPLE 23: 1R trans 3/(△Z) 2-méthyl thio 2-éthoxy carbonyl éthényl) 2,2-diméthyl cyclopropane carboxylate de (S)cyano 1(3-phénoxy phényl) méthyle.**

(1R trans △Z $R_1=CH_3$ $R_2=$

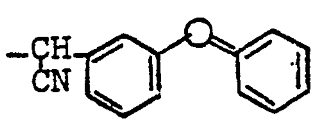

$R_3=C_2H_5$ X=S)

**Stade A**

Acide 1R, trans 3-(△Z 2-méthyl thio 2-éthoxy carbonyl éthényl) 2,2-diméthyl cyclopropane carboxylique

**Stade $A_1$**

1R, trans 3(△Z 2-méthyl thio 2-éthoxy carbonyl éthényl) 2,2-diméthyl cyclopropane carboxylate de terbutyle

Dans 50 $cm^3$ d'éthanol on introduit 2,3 g de sodium, refroidit à 0°C; lorsque l'éthylate est formé,on introduit

9,9 g de 1R, trans 2,2-diméthyl 3-formyl cyclopropane carboxylate de terbutyle, 13,4 g de méthyl thio acétate d'éthyle, agite pendant 16 heures à + 5° C, concentre à sec par distillation sous pression réduite, ajoute de l'eau et de l'éther éthylique, agite, sépare par décantation la phase organique, la lave à l'eau, la concentre à sec par distillation sous pression reduite, chromatographie le résidu sur silice en éluant par un mélange d'hexane et d'acétate d'éthyle (8/2) et obtient 5,6 g de produit attendu.

. Spectre de R. M. N. (deutérochloroforme)
pics à 1,15-1,27-1,38 ppm et 4,05-4,16-4,28-4,4 ppm attribués aux hydrogènes de l'éthyle
pics à 1,18-1,27 ppm attribués aux hydrogènes des méthyles géminés
pic à 1,43 ppm attribué aux hydrogènes des méthyles du terbutyle
pics à 1,67-1,75 ppm attribués à l'hydrogène en position 1 du cyclopropyle
pic à 2,28 ppm attribué aux hydrogènes du méthyle du thio méthyle
pics à 2,53-2,78 ppm attribués à l'hydrogène en position 3 du cyclopropyle
pics à 6,7-6,8 ppm attribués à l'hydrogène éthylénique.

**Stade; $A_2$**

Acide 1R, trans 3-(ΔZ 2-méthyl thio 2-éthoxy carbonyl éthényl) 2,2-diméthyl cyclopropane carboxylique

On porte au reflux pendant 45 minutes 5 g de produit obtenu ci-dessus dans 50 $cm^3$ de toluène en présence de 0,5 g d'acide para toluène sulfonique. On refroidit à 20° C verse dans 100 $cm^3$ d'eau et extrait à l'éther éthylique. Les phases organiques réunies sont séchées et concentrées a sec sous pression réduite. Le résidu est chromatographié sur silice. On élue avec un mélange hexane-acétate d'éthyle (4/6) et obtient 3,47 g de produit attendu.

Spectre de R. M. N. (deutérochloroforme)
pics à 1,27-1,38 ppm attribués aux hydrogènes des méthyles geminés
pics à 2,65-2,74-2,82-2,90 ppm attribués à l'hydrogène en 3 du cyclopropyle
pics à 1,77-1,87 ppm attribués à l'hydrogène en 1 du cyclopropyle
pics à 6,77-6,8 ppm attribués à l'hydrogène éthylénique
pics à 2,28 ppm attribués aux hydrogènes du méthylthio
pics à 1,22-1,33-1,45 et 4,1-4,2-4,3-4,4 ppm attribués aux hydrogènes de l'éthyle.

**Stade B**

1R, trans 3-(ΔZ 2-méthyl thio 2-éthoxy carbonyl éthényl) 2,2-diméthyl cyclopropane carboxylate de (S)cyano 1-(3-phénoxy phényl) méthyle

En opérant comme à l'exemple 2, à partir de l'acide préparé au stade A et de l'alcool approprié, on obtient le produit recherche.

Spectre de R. M. N. (deutérochloroforme)
pics à 1,23-1,3 ppm attribués aux hydrogènes des méthyles géminés
pics à 1,8-1,9 ppm attribués à l'hydrogène en position 1 du cyclopropyle
pic à 2,3 ppm attribué aux hydrogènes du méthyle du méthyl thio
pics à 2,7-2,8-2,9-3,0 ppm attribués à l'hydrogène en position 3 du cyclopropyle
pic à 6,5 ppm attribué à l'hydrogène porté par le carbone en α du CN
pics à 6,7-6,9 ppm attribués à l'hydrogène éthylénique
pics à 7,0-7,6 ppm attribués aux hydrogènes des noyaux aromatiques.

**EXEMPLE 24: 1R trans(3 ΔE ou ΔZ 2-phényl thio 2-éthoxy carbonyl éthényl) 2,2-diméthyl cyclopropane carboxylate de (S) cyano 1(3-phénoxy phényl) méthyle.**

(1R trans ΔE ou ΔZ $R_1$=

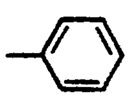

$R_2$=

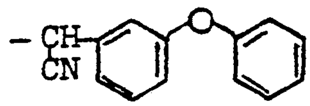

$R_3 = C_2H_5$ X = S)

**Stade A**

Acide 1R trans 3(ΔE ou ΔZ 2-phényl thio 2-éthoxy carbonyl éthenyl) 2,2-diméthyl cyclopropane carboxylique.

**Stade $A_1$**

1R trans 3-(ΔE ou ΔZ 2-phényl thio 2-éthoxy carbonyl éthényl) 2,2-diméthyl cyclopropane carboxylate de terbutyle
(1R, trans ΔE ou ΔZ $R_1$ =

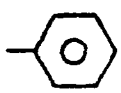

$R_2$ = terbutyle $R_3 = C_2H_5$ X = S)

Dans 50 $cm^3$ d'éthanol on introduit 2,3g de sodium, et introduit, une fois l'éthylate formé, à 0° C, un mélange de 9,9 g de 2,2-diméthyl 3-formyl cyclopropane carboxylate de terbutyle, et de 19,6 g de phénylthio acétate d'éthyle, agite pendant 16 heures à +5° C, élimine le solvant sous pression réduite, ajoute de l'eau et de l'éther éthylique, agite, sépare par décantation la phase etherée, la concentre à sec par distillation sous pression reduite, chromatographie le residu sur silice en éluànt avec un mélange d'hexane et d'acétate d'éthyle (8/2), chromatographie à nouveau dans un mélange d'hexane et d'acétate d'éthyle (9/1) et obtient 5,86 g de produit attendu.

Spectre de R. M. N. (deutérochloroforme)
pics à 0,98-1,1-1,23 ppm et 4,0-4,12-4,23-4,35 ppm attribués aux hydrogènes de l'éthoxy
pics à 1,2-1,23 ppm attribués aux hydrogènes des méthyles géminés
pic à 1,47 ppm attribué aux hydrogènes des méthyles du terbutyle
pics à 1,77-1,85 ppm attribués à l'hydrogène en position 1 du cyclopropyle
pics à 2,57-2,65 ppm et 2,73-2,8 ppm attribués à l'hydrogène en position 3 du cyclopropyle
pics à 6,9-7,1 ppm attribués à l'hydrogène éthylénique
pic à 7,2 ppm attribué aux hydrogènes des noyaux aromatiques.

**Stade $A_2$**

Acide 1R trans 3(ΔE ou ΔZ 2-phényl thio 2-éthoxy carbonyl éthenyl) 2,2 diméthyl cyclopropane carboxylique
(1R, trans ΔE ou ΔZ $R_1$ =

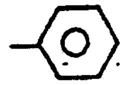

$R_2$ = H $R_3 = C_2H_5$ X = S)

On mélange 5,27 g de 1R trans 3(ΔE ou ΔZ 2-phényl thio 2-éthoxy carbonyl éthényl) 2,2-diméthyl

cyclopropane carboxylate de terbutyle, 50 $cm^3$ de toluène, 0,5 g d'acide para toluène sulfonique, porte le mélange réactionnel au reflux, l'y maintient pendant 45 minutes, refroidit verse dans l'eau, agite, sépare par décantation la phase organique, extrait à l'éther éthylique, réunit les phases organiques, les concentre à sec par distillation sous pression réduite, chromatographie le résidu sur silice en éluant par un mélange d'hexane et d'acétate d'éthyle (6/4) et obtient 3,32 g d'acide attendu.

Spectre de R.M.N. (deutérochloroforme)
pics à 0,98-1,1-1,2 ppm et 3,9-4,05-4,2-4,28 ppm attribués aux hydrogènes de l'éthyle
pic à 1,27 ppm attribué aux hydrogènes des méthyles géminés
pics à 1,8-1,9 ppm attribués à l'hydrogène en position 1 du cyclopropyle
pics à 2,6-2,7-2,8-2,9 ppm attribués à l'hydrogène en position 3 du cyclopropyle
pics à 6,9-7,1 ppm attribués à l'hydrogène éthylénique
pic à 7,2 ppm attribué aux hydrogènes du noyau aromatique
pic à 8,5 ppm attribué à l'hydrogène du carboxyle.

**Stade B**

1R, trans 3-(ΔE ou ΔZ 2-phényl thio 2-éthoxy carbonyl ethényl) 2,2-diméthyl carboxylate de (S)cyano 1(3-phénoxy phényl) méthyle

En opérant comme à l'exemple 2, à partir de l'acide préparé au stade A et de l'alcool approprié, on obtient le produit recherché (F=84° C).

Spectre de R. M. N. (deutérochloroforme)
pics à 1,0-1,11-1,23 ppm et 3,96-4,08-4,2-4,3 ppm attribués aux hydrogènes de l'éthyle
pics à 1,18-1,25 ppm attribués aux hydrogènes des méthyles géminés
pics à 1,89-1,98 ppm attribués à l'hydrogène en position 1 du cyclopropyle
pics à 2,7-2,8 et 2,83-2,9 ppm attribués à l'hydrogène en position 3 du cyclopropyle
pic à 6,4 ppm attribué à l'hydrogène porté par le carbone en α du CH
pics à 6,9-7,7 ppm attribués aux hydrogènes des noyaux aromatiques.

**EXEMPLE 25: 1R, cis 3(-ΔZ 2-phényl thio 2-éthoxy carbonyl éthenyl) 2,2-diméthyl cyclopropane carboxylate de (S) cyano 1(3-phénoxy phényl) méthyle.**

(1R cis ΔZ $R_1$=

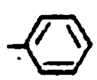

$R_2$=

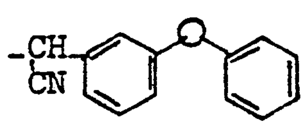

$R_3=C_2h_5$ X=S)

**Stade A**

Acide 1Rcis 3(ΔZ 2-phénylthio 2-éthoxy carbonyl éthényl) 2,2-diméthyl cyclopropane carboxylique

**Stade $A_1$**

Acide 1R, cis 3(1-hydroxy 2-phényl thio 2-éthoxy carbonyl éthyl) 2,2-diméthyl cyclopropane carboxylique

Dans 100 $cm^3$ de tetrahydrofurane on introduit 5,25g de phényl thio acétate d'éthyle, 3,5 g de 1R, 5S 6,6-

diméthyl 4 (R) hydroxy 3-oxa bicyclo/3.1.0./hexane 2-one, ajoute à -60° C, 5,6 g de terbutylate de potassium et 50 $cm^3$ de tétrahydrofurane, agite pendant 1 heure à -60° C, introduit sans dépasser -20° C une solution aqueuse (N) d'acide chlorhydrique, agite, verse dans l'eau, extrait au chlorure de méthylène, lave à l'eau, concentre à sec par distillation sous pression réduite, chromatographie le résidu sur silice en éluant avec un mélange d'hexane et d'acétate d'éthyle (6/4) et obtient 3,5 g d'acide attendu.

Spectre de R. M. N. (deutérochloroforme)
pics à 1,1-1,35 ppm attribués aux hydrogènes des méthyles géminés
pics à 1,1-1,35 ppm attribués aux hydrogènes du méthyle de l'éthyle
pics à 1,11-1,77 ppm attribués aux hydrogènes en position 1 et 3 du cyclopropyle
pics à 3,7-4,66 ppm attribués aux hydrogènes de l'ethyle en 3
pics à 3,7-4,67 ppm attribués aux hydrogènes du methylène de l'éthyle
pics à 5,94 ppm attribues à l'hydrogène de l'hydroxyle
pics à 5,94 ppm attribues à l'hydrogène du carboxyle.

**Stade $A_2$**

6,6-diméthyl 4(1-phenyl thio 1-ethoxy carbonyl methyl) 3-oxa bicyclo /3.1.0./ hexan 2-one

Dans 100 $cm_3$ de benzène on introduit 3,38 g d'acide 1R,cis 3(1-hydroxy 2-phényl thio 2-éthoxy carbonyl éthyl) 2,2-diméthyl cyclopropane carboxylique et 50 mg d'acide para toluène sulfonique porte au reflux, distille environ 30 $cm_3$ de benzène, equipe le ballon d'un appareil Dean-Stark garni de gel de silice,maintient le reflux pendant 1 heure 30 minutes, concentre à sec par distillation sous pression réduite, chromatographie le résidu sur silice en éluant dans un mélange d'hexane et d'acétate d'éthyle (6/4) et obtient 2,6 g de produit attendu.

Spectre de R. M. N. (deutérochloroforme)
pics à 1,11-1,23-1,35 et 4,05-4,16-4,28-4,45 ppm attribués aux hydrogènes de l'éthyle
pics à 1,12-1,2 ppm attribués aux hydrogènes des méthyles géminés
pics à 1,95-2,41 ppm attribués aux hydrogènes en position 1 et 3 du cyclopropyle
pics à 3,7-3,8 ppm attribués à l'hydrogène de

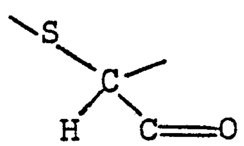

pics à 4,5-4,63 ppm attribués à l'hydrogène de

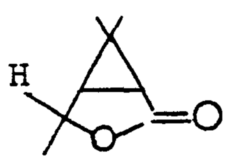

pics à 7,3-7,7 ppm attribués aux hydrogènes du noyau aromatique.

**Stade $A_3$**

Acide 1R, cis 3($\Delta$Z 2-phényl thio 2-éthoxy carbonyl éthényl) 2,2-diméthyl cyclopropane carboxylique (1R, cis $R_1$=

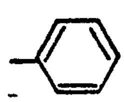

$R_2$=H $R_3$=$C_2H_5$ X=S)

. Dans 22 $cm^3$ de benzène on introduit 2,2 g de 6,6-diméthyl 4(1-phényl thio 1-éthoxy carbonyl méthyl) 3-oxa bicyclo /3.1.0./ hexane 2-one, 2 $cm_3$ de triétylamine, porte le mélange réactionnel au reflux, l'y maintient pendant 3 heures, refroidit à 20° C, introduit une solution aqueuse N d'acide chlorhydrique, sépare la phase organique par décantation, la lave à l'eau, la concentre à sec par distillation sous pression réduite, chromatographie le résidu

sur silice en éluant avec un mélange de chlorure de méthylène et d'acétate d'éthyle (8/2) et obtient 1,1 g d'acide attendu. (F vers 50°C)

Spectre de R. M. N. (deutérochloroforme)
pics à 1,02-1,13-1,25 ppm et 4-4,2-4,23-4,35 ppm attribués aux hydrogènes de l'éthyle
pics à 1,22-1,38 ppm attribués aux hydrogènes des méthyles géminés
pics à 1,9-2,04 ppm attribués à l'hydrogène en position 1 du cyclopropyle
pics à 2,8-3,0 ppm attribués à l'hydrogène en position 3 du cyclopropyle
pic à 7,3 ppm attribué aux hydrogènes du noyau aromatique
pics à 7,7-7,9 ppm attribués à l'hydrogène éthylénique.

**Stade B**

1R, cis 3-(ΔZ 2-phényl thio 2-éthoxy carbonyl éthényl) 2,2-diméthyl cyclopropane carboxylate de (S)cyano 1(3-phénoxy phényl) méthyle

En opérant comme à l'exemple 2, à partir de l'acide et de l'alcool correspondant, on obtient le produit recherché.

Spectre R. M. N. en ppm
1,18-1,27 hydrogène attribués à des méthyles géminés
2,5-2,8 hydrogène en 1 attribué au cyclopropyle
1,92-2,05 hydrogène en 3 attribué au cyclopropyle
7,6-7,8 hydrogène éthylénique
6,48 hydrogène en α du CN
1,03-1,15-1,27 et 4,02-4,13-4,25-4,36 hydrogène de l'éthoxyle
7,3 hydrogènes aromatiques du thio phényle
6,92 à 7,6 hydrogènes aromatiques du phénoxy phényle.

**EXEMPLE 26: (1R, cis)-3(ΔE 2-méthyl thio 2-éthoxy carbonyl éthényl) 2,2-diméthyl cyclopropane carboxylate de (S)cyano 1(3-phénoxy phényl) méthyle.**

(1R, cis ΔE $R_1 = CH_3$ $R_2 =$

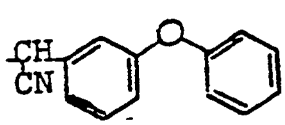

$R_3 = C_2H_5$ $X = S$)

En opérant comme à l'exemple 2, à partir de l'acide ΔE obtenu à l'exemple 22 stade $A_3$ et de l'alcool approprié, on obtient le produit recherché.

Spectre de R. M. N. (deutérochloroforme)
pics à 1,25-1,26 ppm attribués aux hydrogènes des méthyles géminés
pics à 1,25-1,37-1,48 ppm et 4,1-4,2-4,4-4,5 ppm attribués aux hydrogènes de l'éthoxyle
pics à 1,85-2,0 ppm attribués à l'hydrogène en position 1 du cyclopropyle
pics à 2,25 ppm attribués aux hydrogènes du thio méthyle
pics à 2,8-3,2 ppm attribués à l'hydrogène en position 3 du cyclopropyle
pics à 6,1-6,3 ppm attribués à l'hydrogène ethylénique
pic à 6,4 ppm attribué à l'hydrogène porté par le carbone en α du CN
pics à 6,9-7,6 ppm attribués aux hydrogènes des noyaux aromatiques.

**EXEMPLE 27: (1R,trans) 2,2-diméthyl 3-(ΔZ 2-méthylsulfinyl 2-méthoxycarbonyléthényl) cyclopropane carboxylate de (S) cyano (3-phénoxyphényl) methyle - diastéréoisomère A et diastéréoisomère B.**

Dans 200 $cm^3$ de méthanol, on introduit 6 g de 1R, trans 3-(ΔE + ΔZ 2-méthylthio 2-méthoxycarbonyléthényl) 2,2-diméthyl cyclopropane carboxylate de (α) (S) cyano 1-(3-phénoxyphényl) méthyle, 5,7 g de métapériodate de sodium en solution dans 60 $cm^3$ d'eau, agite à 20°C pendant 24 heures, ajoute 1,8 g de métapériodate de sodium, agite pendant 18 heures à 20°C, filtre, concentre le filtrat à sec par distillation sous pression réduite, extrait à

l'acétate d'éthyle, concentre à sec par distillation sous pression réduite, chromatographie sur silice en éluant par un mélange de cyclohexane et d'acétate d'éthyle (1/1) et obtient 0,5 g de diastéréoisomère A et 0,5 g de diastéréoisomère B.

**Diastereoisomère A**:

/α/$_D$ = +152,5° (c = 1% benzène)

**Diastéréoisomère B**:

/α/$_D$ = +57,5° (c = 0,3% benzène)

Le 1R, trans 3-(ΔE + ΔZ 2-méthylthio 2-méthoxycarbonyléthényl) 2,2-diméthyl cyclopropane carboxylate de (S) cyano 1-(3-phénoxyphényl) méthyle utilisé au départ de l'exemple 27 peut être prépare comme suit:

**Stade A: acide 1R, trans 3-/(ΔE + ΔZ) 2-méthylthio 2-méthoxycarbonyléthényl/ 2,2-diméthyl cyclopropane carboxylique.**

Dans 100 $cm^3$ de méthanol, on introduit 14,2 g d'acide 1R, trans 2,2-diméthyl 3-formyl cyclopropane 1-carboxylique, 13,2 g de méthylthio acétate de méthyle, ajoute à +10° C une solution de 14 g de méthylate de potassium dans 100 cm3 de méthanol, agite pendant 18 heures à 20° C, concentre à sec par distillation sous pression réduite, chromatographie sur silice en éluant par un mélange de cyclohexane et d'acétate d'éthyle (1/1) et obtient 5,2 g de produit recherché.

**Stade B: 1R,trans 3-(ΔZ + ΔE 2-méthylthio 2-méthoxycarbonyléthényl) 2,2-diméthyl cyclopropane carboxylate de (S) cyano 1-(3-phénoxyphényl) méthyle.**

Dans 70 $cm^3$ de chlorure de méthylène, on introduit 5,1 g d'acide obtenu au stade A, ajoute 4,26 g de dicyclohexanecarbodiimide, 1,95 $cm^3$ de pyridine, agite pendant 15 minutes à 20° C, introduit 4,7 g d'alcool (α)(S) cyano 1-(3-phénoxyphényl) méthylique, agite pendant 18 heures à 20° C, filtre, concentre le filtrat à sec par distillation sous pression réduite, chromatographie le résidu sur silice en éluant au benzène et obtient 6,4 g de produit recherché.

**EXEMPLE 28: 1R,trans 3-(Δ E 2-méthoxy 2-isopropyloxy carbonyl éthényl) 2,2-diméthyl cyclopropane carboxylate de (S) cyano 1(3-phénoxyphényl) méthyle.**

Dans 15 $cm^3$ de chlorure de méthylène, on dissout 1,5 g d'acide 1R,trans 3-(ΔE 2-méthoxy 2-isopropyloxy carbonyléthényl) 2,2-diméthyl cyclopropane carboxylique (préparé de manière analogue à celle décrite à l'exemple 16 pour l'isomere ΔZ) et 1,19 g d'alcool α(S) cyano 1-(3-phénoxyphényl) méthylique, introduit à 0° C 15 cm3 d'une solution chlorométhylénique contenant 1,26 g de dicyclohexylcarbodiimide et 100 mg de 4-diméthylamino pyridine. On agite pendant 16 heures à température ambiante, essore l'urée formée, rince au chlorure de méthylène et concentre à sec le filtrat sous pression réduite. On obtient 2,95 g de produit brut que l'on chromatographie sur silice, élue par un mélange hexaneacétate d'éthyle (8/2). On recueille 1,58 g de produit attendu.

/α/$_D$ = +2° (c = 0,5% chloroforme)

**EXEMPLE 29: 1R,trans 3-(ΔZ 2-méthoxy 2-méthoxycarbonyléthényl) 2,2-diméthyl cyclopropane carboxylate de 1-(2-pyridyl 3-phénoxy) méthyle.**

D'une manière analogue à celle de l'exemple 28, au départ de l'acide 1R,trans 3-(ΔZ 2-méthoxy 2-méthoxycarbonyléthényl) 2,2-diméthyl cyclopropane carboxylique et de l'alcool 1-(2-pyridyl 3-phénoxy) méthylique, on obtient le produit recherché.

/α/$_D$ = +22° (c = 1,2% chloroforme)

**EXEMPLE 30: 1R,cis 2,2-diméthyl 3-( Δ E 2-méthylsulfinil 2-méthoxycarbonyléthényl) cyclopropane carboxylate de (S) cyano (3-phénoxyphényl) méthyle - diastéréoisomere A et diastéréoisomère B).**

Dans 70 $cm^3$ de chlorure de méthylene, on introduit 6,1 g de 6,6-diméthyl 4-(méthoxycarbonyl) (méthylsulfinyl) méthyl 3-oxa-bicyclo /3.1.0./ hexane-2-one, 2,1 $cm^3$ de pyridine, ajoute 4,76g de dicyclohexylcarbodiimide, agite pendant 15 minutes, introduit 5,26 g d'alcool α(S) cyano (3-phénoxyphényl) méthylique, agite pendant 18 heures à 20° C, filtre, concentre le filtrat à sec par distillation sous pression réduite, chromatographie sur silice en éluant par un mélange de cyclohexane et d'acétate d'éthyle (6/4) et obtient 2,6 g de diastéréoisomère A et 2,2 g de diastéréoisomère B.

**Diastéréoisomère A**:

/α/D = +356° (c = 0,3% benzène)

**Diastéréoisomère B**:

$/\alpha/_D$ = -9,5° (c = 1% benzène)

Le 6,6-diméthyl 4-(méthoxycarbonylsulfinylméthyl) 3-oxabicyclo /3.1.0./ hexane-2-one utilisé au départ de l'exemple peut être préparé comme suit.

On dissout 5,68 g de 1R,5S 6,6-diméthyl 4(R)-hydroxy 3-oxa-bicyclo /3.1.0./ hexane 2-one dans 50 $cm^3$ de chlorure de méthylène, ajoute 5,44 g de méthylsulfinyl acétate de méthyle, 3,4$cm^3$ de pyrrolidine, agite pendant 18 heures à 20° C, lave par une solution aqueuse N d'acide chlorhydrique, concentre à sec par distillation sous pression réduite, chromatographie le résidu sur silice en éluant par un mélange d'acétate d'éthyle et d'acide acétique (100/2) et obtient 6,1 g de produit recherché.

**EXEMPLE 31: 1R trans 2,2-diméthyl 3-( Δ E 2-méthylsulfinyl 2-méthoxycarbonyléthényl) cyclopropane carboxylate de (S) cyano (3-phénoxyphényl) méthyle - diastéréoisomere A et diastéréoisomère B.**

Dans 60 $cm^3$ de chlorure de méthylène, on introduit 5,6 g d'acide 1R,trans 2,2-diméthyl 3-( Δ E 2-méthylsulfinyl 2-méthoxy carbonyléthényl) cyclopropane carboxylique, 1,95 $cm^3$ de pyridine, 4,4 g de dicyclohexylcarbodiimide, agite pendant 15 minutes à 20° C, ajoute 4,84 g d'alcool α (S) cyano (3-phénoxyphényl) méthylique, agite pendant 16 heures à 20° C, filtre, concentre le filtrat à sec par distillation sous pression réduite, chromatographie le résidu sur silice en éluant avec un mélange de cyclohexane et d'acétate d'éthyle (6/4) et obtient 1,0 g de diastéréoisomère A et 1,1 g de diastéréoisomere B.

**Diastéréoisomère A**

/α/D = +26,5° (c = 0,5% benzène)

**Diastéréoisomère B**

$/\alpha/_D$ = +130° (c = 0,5% benzène)

L'acide 1R,trans 2,2-diméthyl 3-( Δ E 2-méthylsulfinyl 2-méthoxycarbonyléthényl) cyclopropane carboxylique utilisé au départ de l'exemple 31 peut être préparé comme suit.

On dissout 5,44 g de méthylsulfinyl acétate de méthyle, 5,68 g d'acide 1R,trans 2,2-diméthyl 3-formyl cyclopropane carboxylique dans 50 cm3 de chlorure de méthylene, ajoute 3,4 $cm^3$ de pyrrolidine, agite pendant 18 heures à 20° C, ajoute du chlorure de méthylène, lave par une solution aqueuse N d'acide chlorhydrique, concentre à sec par distillation sous pression réduite, chromatographie le résidu sur silice en éluant avec un mélange d'acétate d'éthyle et d'acide acétique (100/2) et obtient 6,2 g de produit recherché.

$/\alpha/_D$ = +106,5° (c = 1% benzène)

**EXEMPLE 32: (1R, cis) 2,2-diméthyl 3-( △Z 2-méthylsulfinyl 2-méthoxycarbonyléthényl) cyclopropane carboxylate de (S) cyano (3-phénoxyphényl) méthyle - Disastéréoisomère A et diastéréoisomère B.**

De manière analogue à celle de l'exemple 27, au départ de (1R,cis) 3-( △Z 2-méthylthio 2-méthoxycarbonyléthényl) 2,2-diméthyl cyclopropane carboxylate de (S) cyano 1-(3-phénoxyphényl) méthyle, on obtient le produit recherché sous forme de diastéréoisomère A et de diastéréoisomère B.

**Diastéréoisomère A**

/α/$_D$ = +73° (c = 1% benzène)

**Diastéréoisomère B**

/α/$_D$ = +52° (c = 1% benzène)

Le 1R,cis 3-( △Z 2-méthylthio 2-méthoxycarbonyléthényl 2,2-diméthyl cyclopropane carboxylate de (S) cyano 1-(3-phénoxyphényl) méthyle utilisé au départ de l'exemple 34 peut être préparé comme suit.

**Stade A: Acide (1R,cis) 3-( △Z 2-méthylthio 2-méthoxycarbonyléthényl) 2,2-diméthyl cyclopropane carboxylique.**

Dans 75 $cm^3$ de tétrahydrofurane, on dissout 5,68 g de 1R, 5S 6,6-diméthyl 4(R)-hydroxy 3-oxabicyclo /3.1.0./ hexane-2-one et 5,28 g de méthylthioacétate de méthyle, introduit lentement à -60° C une solution de 8,97 g de terbutylate de potassium dans 50 $cm^3$ de tétrahydrofurane, agite pendant 1 heure à -60° C, ajoute une solution saturée de phosphate monosodique, extrait à l'acétate d'éthyle concentre à sec par distillation sous pression réduite dissout le résidu dans le benzène ajoute 100 mg d'acide paratoluène sulfonique, porte au reflux pendant 1 heure 30 minutes, élimine l'eau formé par distillation azéotropique, ajoute 12 cm3 de triéthylamine, porte le mélange réactionnel au reflux et l'y maintient pendant 3 heures, concentre à sec par distillation sous pression réduite, chromatographie le résidu sur silice en éluant par un mélange de cyclohexane et d'acétate d'éthyle (1/1) et obtient 6,3 g de produit recherché contenant d'après le spectre de RMN 10% d'isomère △E.

Spectre de RMN (deutérochloroforme)
pics à 1,32-1,35 ppm des hydrogènes des méthyles géminés
pics à 1,9-2,1 ppm de l'hydrogène en position 1 du cyclopropane
pic à 2,23 ppm des hydrogènes du thiométhyle
pics à 2,52-2,83 ppm de l'hydrogène en position 3 du cyclopropane
pic à 3,85 ppm des hydrogènes du méthoxy
pics à 6,3-6,5 ppm de l'hydrogène éthylénique (~1/10 de △E)
pics à 7,4-7,6 ppm de l'hydrogène éthylénique (~ 9/10 de △Z)

**Stade B: 1R,cis 3-( △Z 2-méthylthio 2-méthoxycarbonyléthényl) 2,2-diméthyl cyclopropane carboxylate de (S) cyano 1-(3-phénoxy phényl) méthyle.**

Dans 75 $cm^3$ de chlorure de méthylène, on dissout 6,3 g d'acide ( △Z + △ E) préparé au stade A, ajoute 5,26 g de dicyclohexylcarbodiimide, agite à 20° C pendant 15 minutes, ajoute 5,8 g d'alcool α(S) cyano 1-(3-phénoxyphényl) méthylique et 2,4 $cm^3$ de pyridine, agite pendant 18 heures à 20° C, filtre, concentre le filtrat à sec par distillation sous pression réduite, chromatographie le résidu sur silice en éluant par un mélange de cyclohexane et d'acétate d'éthyle (9/1) et obtient 6,8 g de l'isomère △Z du produit recherché et 0,8 g de l'isomère △E du produit recherché.

**Isomère △Z**

Dichroïsme circulaire (dioxane)
max. à 257 nm, $\Delta\varepsilon$ = +1,15; max. à 290 nm, $\Delta\varepsilon$= +1,0; max. à 305 nm, $\Delta\varepsilon$ = +0,9.

**Isomère △E**

max. à 240 nm, △ε -0,6; max. à 275 nm, △ε = -0,25; max. à 300 nm, △ε = -0,6.

**EXEMPLE 33: (1R,trans) 3-( △ E 2-méthoxy 2-méthoxycarbonyléthényl) 2,2-diméthyl cyclopropane carboxylate de (6-phénoxy 2-pyridyl) méthyle.**

De manière analogue à celle de l'exemple 28, au départ de l'acide (1R,trans) 3-( △ E2-méthoxy 2-méthoxycarbonyléthényl) 2,2-diméthyl cyclopropane carboxylique et d'alcool (6-phénoxy 2-pyridyl) méthylique, on obtient le produit recherché.
$/\alpha/_D$ = +9° (c = 0,7% chloroforme)

**EXEMPLE 34: (1R,trans 3-( △ Z 2-méthoxy 2-terbutoxycarbonyléthényl) 2,2-diméthyl cyclopropane carboxylate de (S) cyano 1-(3-phénoxy phényl) méthyle.**

De manière analogue à celle décrite dans l'exemple 28, au départ d'acide (1R,trans) 3-( △Z 2-méthoxy 2-terbutoxycarbonyléthényl) 2,2-diméthyl cyclopropane carboxylique et de l'alcool α (S) cyano 1-(3-phénoxyphényl) méthylique, on obtient le produit recherché. F = 72°C.
$/\alpha/_D$ = -90° (c = 0,7% chloroforme)
L'acide 1R,trans 3-( △Z 2-méthoxy 2-terbutoxycarbonyléthényl) 2,2-diméthyl cyclopropane carboxylique utilisé au départ de l'exemple 37 peut être préparé comme suit.

**Stade A: 1R,trans 3-( △Z 2-méthoxy 2-terbutoxycarbonyléthényl) 2,2-diméthyl cyclopropane carboxylate de méthoxy méthyle - Isomère E et isomère Z.**

On mélange 0,88 g de terbutylate de potassium, 4 $cm^3$ d'alcool terbutylique, 2 cm3 de diméthylformamide, 4 $cm^3$ de tétrahydrofurane, introduit à 0°C, 2,13 g de 0,0-diéthyle phosphono terbutoxy acétate de méthyle en solution dans 3 $cm^3$ de tétrahydrofurane, agite pendant 30 minutes, introduit à 0°C, une solution de 1R,trans 2,2-diméthyl 3-formyl cyclopropane carboxylate de méthoxy méthyle dans 6 $cm^3$ de tétrahydrofurane, agite pendant 1 heure à 0°C, verse le mélange réactionnel dans l'eau et extrait à l'éther isopropylique, agite, décante, concentre à sec la phase organique par distillation sous pression réduite, chromatographie le résidu sur silice en éluant avec un mélange de benzène et d'acétate d'éthyle (95/5) et obtient 0,97 g d'isomère △Z et 0,45 g d'isomère △E.

**Isomère △Z**

**Spectre de RMN** (deutérochloroforme)

pics à 1,25-1,32 ppm des hydrogènes des méthyles géminés
pic à 1,32 ppm des hydrogènes des méthyles du terbutyle
pics à 1,66-1,76 ppm de l'hydrogène en position 1 du cyclopropane
pics de 2,4 à 2,65 ppm de l'hydrogène en position 3 du cyclopropane
pic à 3,5 ppm de l'hydrogène du méthyle du méthoxyméthyle
pic à 3,75 ppm des hydrogènes du méthyle du méthoxycarbonyle
pic à 5,25 ppm des hydrogènes du méthylène du méthoxymethyle
pics à 6,0-6,2 ppm de l'hydrogène éthylénique

**Isomère △E**

**Spectre de RMN** (deutérochloroforme)

pics à 1,18-1,32 ppm des hydrogènes des méthyles géminés
pic à 1,25 ppm des hydrogènes des méthyles du terbutyle
pics à 1,5-1,6 ppm de l'hydrogène en position 1 du cyclopropane
pics de 2,68 à 2,93 ppm de l'hydrogène en position 3 du cyclopropane
pic à 3,5 ppm des hydrogènes du méthyle du méthoxyméthyle
pic à 3,8 ppm des hydrogènes du méthyle du méthoxycarbonyle
pic à 5,25 ppm des hydrogènes du méthylène du méthoxyméthyle
pics à 5,4-5,5 ppm de l'hydrogène éthylénique.

**Stade B: Acide 1R,trans 3-(ΔZ 2-méthoxy 2-terbutoxycarbonyléthenyl) 2,2-diméthyl cyclopropane carboxylique.**

On mélange 910 mg de 1R,trans 3-(ΔZ 2-méthoxy 2-terbutoxy carbonyléthényl) 2,2-diméthyl cyclopropane carboxylate de méthoxy méthyle, 17,5 cm3 de méthanol, 17,5 cm3 d'acétone, 35 $cm^3$ de solution aqueuse N d'acide chlorhydrique, agite pendant 6 heures et 30 minutes à 20° C, verse le mélange réactionnel dans l'eau, extrait à l'éther éthylique, concentre à sec par distillation sous pression réduite, chromatographie le résidu sur silice en éluant avec un mélange d'hexane et d'acétate d'éthyle (4/6) et obtient 0,47 g de produit recherché.

**Spectre IR** (éthanol)

OH acide monomère + dimère 3500 $cm^{-1}$

$C=O$ ester 1725 $cm^{-1}$

$C=O$ acide 1695 $cm^{-1}$

C=C ΔZ 1640 $cm^{-1}$
bande COOMe 1440 $cm^{-1}$
t-Bu 1372 $cm^{-1}$

**EXEMPLE 35: 1R,trans 3-(ΔZ 2-terbutoxy 2-terbutoxycarbonyléthényl) 2,2-diméthyl cyclopropane carboxylate de (S) cyano 1-(3-phénoxyphényl) méthyle.**

De manière analogue à celle utilisée à l'exemple 28, au départ d'acide 1R,trans 3-(ΔZ 2-terbutoxy 2-terbutoxycarbonyléthényl) 2,2-diméthyl cyclopropane carboxylique et d'alcool α (S) cyano 1-(3-phénoxyphényl) méthylique, on obtient le produit recherché. F = 81°C.
$/\alpha/_D$ = -9° (c = 0,5% chloroforme)
L'acide 1R,trans 3-(ΔZ 2-terbutoxy 2-terbutoxycarbonyléthé-nyl) 2,2-diméthyl cyclopropane carboxylique utilisé au départ de l'exemple 35 peut être préparé comme suit.

**Stade A: 1R,trans 2,2-diméthyl 3-formyl cyclopropane carboxylate de méthoxyméthyle.**

Dans 250 $cm^3$ de tétrahydrofurane, on dissout 25,6 g d'acide 1R,trans 2,2-diméthyl 3-formyl cyclopropane carboxylique, introduit 1,64 g d'hydrure de lithium, agite pendant 30 minutes à +15°C, introduit 18,2 g de solution franchement préparée de chlorométhyl méthyléther (0,225 mole), agite pendant 4 heures à 20°C, verse le mélange réactionnel dans un mélange d'eau, de glace et de bicarbonate de sodium, agite, extrait à l'éther

isopropylique, concentre la phase organique à sec par distillation sous pression réduite, rectifie sous vide et obtient 15,15 g de produit recherché. eb = 67° C sous 0,05 mm de mercure.

**Stade B: 1R,trans 3-( △Z 2-terbutoxy 2-terbutoxycarbonyléthényl) 2,2-diméthyl cyclopropane carboxylate de méthoxy méthyle.**

On mélange 2,8 g de terbutylate de potassium, 13,3 $cm^3$ de terbutanol, 6,6 cm3 de diméthylformamide, 13,3 $cm^3$ de tétrahydrofurane, introduit à 0° C une solution de 8,1 g de 0,0-diéthyl phosphono terbutoxy acétate de terbutyle dans 13,3 cm3 de tétrahydrofurane, agite pendant 30 minutes à 0° C, introduit une solution de 4,66 g de 1R,trans 2,2-diméthyl 3-formyl cyclopropane carboxylate de méthoxyméthyle dans 20 $cm^3$ de tétrahydrofurane, agite pendant 1 heure à -5° C, verse le mélange réactionnel dans l'eau, extrait à l'éther isopropylique, concentre la phase organique à sec par distillation sous pression réduite, chromatographie le résidu sur silice en éluant avec un mélange d'hexane et d'acétate d'éthyle (8/2) et obtient 0,59 g de produit recherché.

**Stade C: Acide 1R,trans 3-( △Z 2-terbutoxy 2-terbutoxycarbonyléthényl) 2,2-diméthyl cyclopropane carboxylique.**

On mélange 590 mg de 1R,trans 3-( △Z 2-terbutoxy 2-terbutoxy carbonyléthényl) 2,2-diméthyl cyclopropane carboxylate de méthoxy méthyle, 20 $cm^3$ de méthanol, 10 $cm^3$ d'acétone, 20 $cm^3$ de solution aqueuse N d'acide chlorhydrique, agite pendant 6 heures à 20° C, verse le mélange réactionnel dans l'eau, extrait au chlorure de méthylène, concentre la phase organique à sec par distillation sous pression réduite, chromatographie le résidu sur silice en éluant par un mélange d'hexane et d'acétate d'éthyle (4/6) et obtient 364 mg de produit recherché. F = 85° C.

**EXEMPLE 36: (1R,trans) ( △E 2-terbutoxy 2-méthoxycarbonyléthényl) 2,2-diméthyl cyclopropane carboxylate de (S) cyano 1-(3-phénoxy phényl) méthyle.**

D'une façon analogue à celle utilisée dans l'exemple 28, au départ d'acide 1R,trans 3-( △E 2-terbutoxy 2-méthoxycarbonyléthényl) 2,2-diméthyl cyclopropane carboxylique et d'alcool α (S) cyano 1-(3-phénoxyphényl) méthylique, on obtient le produit recherché. F = 80° C.
$/\alpha/_D = +7°$ (c = 1% chloroforme)
L'acide 1R,trans 3-( △E 2-terbutoxy 2-méthoxycarbonyléthényl) 2,2-diméthyl cyclopropane carboxylique utilisé au départ de l'exemple 39 peut être préparé comme suit.

On mélange 410 mg de 1R,trans 3-( △E 2-terbutoxy 2-méthoxy carbonyléthényl) 2,2-diméthyl cyclopropane carboxylate de méthoxy méthyle (obtenu à l'exemple 37), 8 $cm^3$ de méthanol, 8 $cm^3$ d'acétone, 16 $cm^3$ de solution aqueuse N d'acide chlorhydrique, agite pendant 6 heures à 20° C, verse dans l'eau, extrait à l'éther éthylique, concentre la phase organique à sec par distillation sous pression réduite et obtient 300 mg de produit recherché.

**EXEMPLE 37: préparation d'un concentré soluble**

On effectue un mélange homogène de:
- produit de l'exemple 7 0,25 g
- butoxyde de pipéronyle 1 g
- twéen 80 0,25 g
- topanol A 0,1 g
- eau 98,4 g

**EXEMPLE 38: préparation d'un concentré émulsifiable**

On mélange intimement:
- produit de l'exemple 39 0,015 g
- butoxyde de pipéronyle 0,5 g
- topanol A 0,1 g
- xylène 99,385 g

**EXEMPLE 39: préparation d'un concentré émulsifiable**

On effectue un mélange homogène de:
- produit de l'exemple 9 1,5 g
- twéen 80 20 g
- topanol A 0,1 g
- xylène 78,4 g

**EXEMPLE 40: préparation d'une composition fumigène**

- produit de l'exemple 3 0,25 g
- poudre de tabu 25 g
- poudre de feuille de cèdre 40 g
- poudre de bois de pin 33,75 g
- vert brillant 0,5 g
- p-nitrophénol 0,5 g

**Etude de l'activité biologique des composés de formule (I). Effet d'abattage sur mouche domestique.**

Les insectes tests sont des mouches domestiques femelles âgées de 4 jours. On opère par pulvérisation directe à la concentration de 0,25 g/l en chambre de Kearns et March en utilisant comme solvant un mélange d'acétone (5 %) et d'Isopar L® (solvant pétrolier) (quantité de solvant utilisée 2 ml en une seconde). On utilise 50 insectes par traitement. On effectue les contrôles toutes les minutes jusqu'à 10 minutes, puis à 15 minutes et l'on détermine le KT 50 par les méthodes habituelles.

Les résultats expérimentaux obtenus sont résumés dans le tableau suivant:

| Composés de l'exemple | KT 50 en minutes |
|---|---|
| 3 | 1,2 |
| 6 | 3,5 |
| 7 | 3 |
| 8 | 2,8 |
| 9 | 2,2 |
| 10 | 5,0 |
| 11 | 1,1 |
| 15 | 2,1 |
| 18 | 3 |
| 19 | 2,5 |
| 28 | 8,45 |
| 29 | 8,33 |
| 30 (Stéréoisomère B) | 8,1 |
| 32 | 7,5 |
| 32 (Stéréoisomère A) | 6,2 |
| 36 | 3,0 |

**Revendications**

1. Sous toutes leurs formes stéréoisomères ou sous forme de mélanges de stéréoisomères, les composés de formule générale (I):

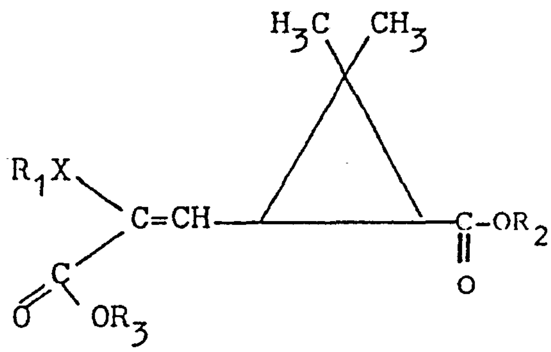

(I)

formule dans laquelle X représente un atome d'oxygène ou de soufre, un groupement sulfoxyde, et <u>ou bien $R_1$</u> et $R_3$ identiques ou différents représentent des radicaux alkyles linéaires, ramifiés ou cyclisés, saturés ou insaturés, et dont la chaîne peut être interrompue par un ou plusieurs hétéro-atomes, ou substituée par un ou plusieurs atomes d'halogènes

<u>ou bien $R_1$</u> et $R_3$ identiques ou différents représentent un radical aryle ou aralkyle renfermant de 6 à 18 atomes de carbone et $R_2$ représente le reste d'un alcool $R_2OH$ utilisé dans la synthèse des pyréthrinoïdes

2. Les composés de formule I selon la revendication 1, dans lesquels X représente un atome d'oxygène.

3. Les composés de formule I selon la revendication 1, dans lesquels X représente un atome de soufre.

4. Les composés de formule I selon l'une quelconque des revendications 1, 2 ou 3, dans lesquels $R_2$ représente l'un des radicaux suivants

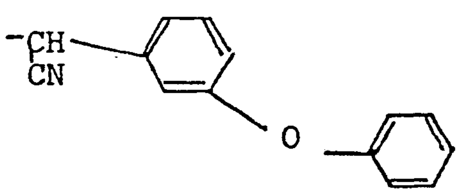

,

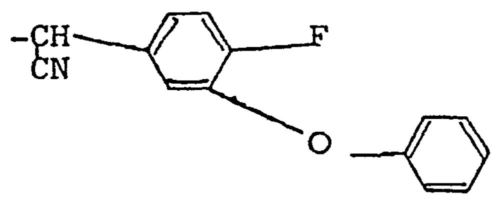

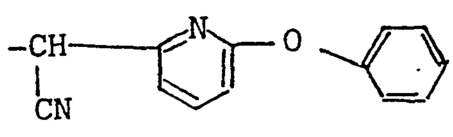

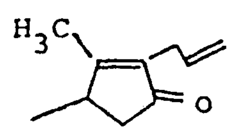

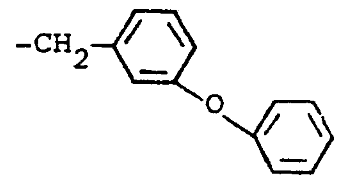

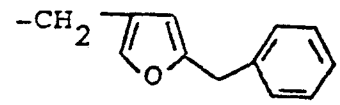

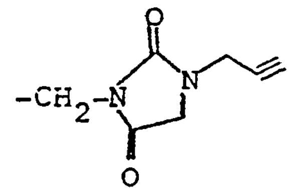

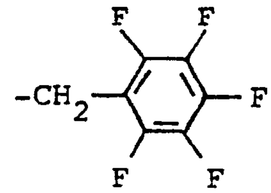

5. Les produits répondant à la formule (I) de la revendication 1 dont les noms suivent:
- le 1R,trans 3-( ΔZ 2-méthoxy 2-méthoxycarbonyléthényl) 2,2)diméthyl cyclopropane carboxylate de (S) -cyano 1-(3-phénoxyphényl) methyle;
- le 1R,trans 3-( ΔZ 2-éthoxy 2-éthoxycarbonyléthényl) 2,2-diméthyl cyclopropane carboxylate de (S) -cyano 1-(3-phénoxyphényl) méthyle;
- le 1R,trans 3-( ΔZ 2-méthoxy 2-méthoxycarbonyléthényl) 2,2-diméthyl cyclopropane carboxylate de 3'-allyl 2'-méthyl 4'-oxo 2'-cy-clopenten-1'-yle;
- le 1R,trans 3-( ΔE 2-méthoxy 2-méthoxycarbonyléthényl) 2,2-diméthyl cyclopropane carboxylate de 3'-allyl 2'-méthyl 4'-oxo 2'-cy-clopenten-1'-yle;
- le 1R,trans 3-( ΔZ 2-méthoxy 2-méthoxycarbonyléthényl) 2,2-diméthyl cyclopropane carboxylate de /1-(3-propyn-2-yl) 2,5-dioxo imidazolidinyl/ méthyle;
- le 1R,trans 3-/( ΔZ ou ΔE) 2-méthoxy 2-méthoxycarbonyléthényl/ 2,2-diméthyl cyclopropane parboxylate de (S) cyano 1-(3-phénoxy 4-fluorophényl) méthyle;
- le 1R,trans 3-/( ΔZ ou ΔE) 2-méthoxy 2-méthoxyéthényl/2,2-diméthyl cyclopropane carboxylate de (RS)cyano 6'-phénoxy 2'-pyridyl méthyle;
- le 1R,trans 3-( ΔZ 2-méthoxy 2-éthoxycarbonyléthényl) 2,2-diméthyl cyclopropane carboxylate de (S) cyano 1-(3-phénoxyphényl) méthyle;
- le 1R,trans 3-( ΔZ 2-méthoxy 2-méthoxycarbonyléthényl) 2,2-diméthyl cyclopropane carboxylate de (S) cyano 1-(3-phénoxyphényl) méthyle;
- le 1R,cis 3-( ΔZ 2-méthoxy 2-méthoxycarbonyléthényl) 2,2-diméthyl cyclopropane carboxylate de (S) cyano 1-(3-phénoxyphényl) méthyle;
- le 1R,cis 3-( ΔZ 2-méthylsulfinyl 2-méthoxycarbonyléthényl) 2,2-diméthyl cyclopropane carboxylate de (S) cyano 1-(3-phénoxy-phényl) méthyle (stéréoisomères A et B);
- le 1R,trans 3-( ΔE 2-terbutoxy 2-méthoxycarbonyléthényl) 2,2-diméthyl cyclopropane carboxylate de (S) cyano 1-(3-phénoxyphe-nyl) méthyle.

6. Procédé de préparation des composés de formule générale (I) tels que définis à la revendication 1, caractérisé en ce que l'on fait réagir un composé de formule (II):

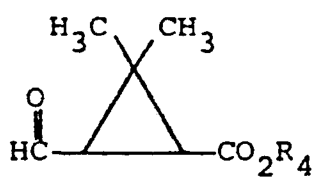

(II)

dans laquelle $R_4$ représente un atome d'hydrogène ou le reste d'un alcool facilement clivable, pouvant se présenter dans le cas où $R_4$ est un atome d'hydrogène et la configuration est cis, sous la forme cyclisée

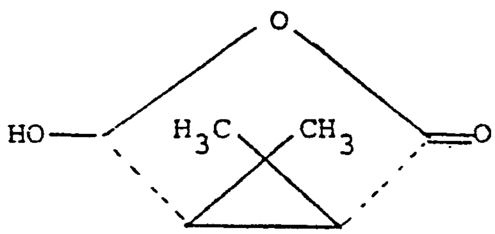

avec un composé de formule (III)

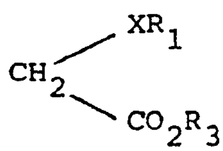

(III)

dans laquelle X, $R_1$ et $R_3$ sont définis comme dans la revendication 1, pour obtenir un composé de formule (IV):

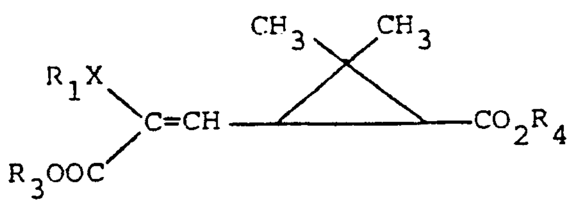

(IV)

que l'on soumet dans le cas où $R_4$ représente un reste d'ester facilement clivable, à l'action d'un agent d'hydrolyse, pour obtenir le composé de formule (IV) dans laquelle $R_4$ représente un atome d'hydrogène, puis soumet le composé de formule (IV) dans laquelle $R_4$ représente un atome d'hydrogène à l'action d'un alcool $R_2OH$ ou de l'un de ses dérivés pour obtenir le composé de formule (1) correspondant.

7. Variante du procédé selon la revendication 6, caractérisée en ce que pour la préparation du composé de formule (IV) l'on soumet le composé de formule (II) à l'action d'un composé de formule (V):

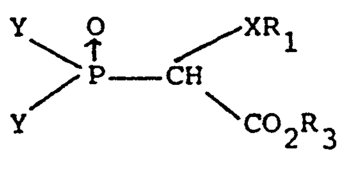

(V)

dans laquelle X, $R_1$ et $R_3$ conservent la même signification que dans la revendication 1 et Y représente un radical alcoxyle ou aryle renfermant jusqu'à 8 atomes de carbone.

8. Variante du procédé selon la revendication 6 ou 7, pour la preparation des composés de formule (I) dans laquelle X représente un groupement sulfoxyde, caractérisée en ce que l'on utilise au départ un composé de formule (III) ou (V) dans laquelle X représente un atome de soufre et que l'on effectue l'oxydation au niveau du composé de formule (I) ainsi obtenu, dans lequel X représente un atome de soufre.

9. A titre de produits industriels nouveaux, les acides de formule générale (IV,)

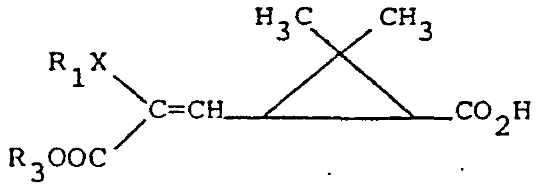

(IV')

formule dans laquelle $R_1$, $R_3$ et X conservent les significations de la revendication 1.

10. A titre de produits tels que définis à la revendication 9, les acides de formule (IV') dont les noms suivent:

- l'acide 1R trans 3-( △E 2-méthoxy 2-méthoxy carbonyl éthényl) 2,2-diméthyl cyclopropane carboxylique et l'acide 1R, trans 3-( △Z 2-méthoxy 2-méthoxy carbonyléthényl) 2,2-diméthyl cyclopropane carboxylique.
- l'acide 1R trans 3-( △Z 2-méthyl thio 2-éthoxy carbonyl éthényl) 2,2-diméthyl cyclopropane carboxylique;
- l'acide 1R,trans 3-( △Z ou △E 2-phénylthio 2-éthoxy carbonyl éthényl) 2,2-diméthyl cyclopropane carboxylique;
- l'acide 1R trans 3-( △E 2-éthoxy 2-éthoxy carbonyl éthényl) 2,2-diméthyl cyclopropane carboxylique et l'acide 1R trans 3-( △Z 2-éthoxy 2-éthoxy carbonyl éthényl) 2,2-diméthyl cyclopropane carboxylique;
- l'acide 1R,cis 3( △Z 2-phényl thio 2-éthoxy carbonyl éthényl) 2,2-diméthyl cyclopropane carboxylique;
- l'acide 1R,cis 3( △E 2-méthoxy 2-méthoxycarbonyl éthényl) 2,2-diméthyl cyclopropane carboxylique et l'acide 1R, cis △Z correspondant;
- l'acide 1R,cis 3-( △Z 2-méthylthio 2-méthoxycarbonyléthényl) 2,2-diméthyl cyclopropane carboxylique et l'acide △E correspondant;
- l'acide 1R,trans 3-( △E 2-méthoxy 2-terbutoxycarbonyléthényl) 2,2-diméthyl cyclopropane carboxylique et l'acide △Z correspondant.

11. Application des composés de formule I tels que définis à l'une quelconque des revendications 1 à 5 à la lutte contre les parasites des végétaux, les parasites des locaux et les parasites des animaux à sang chaud.

12. Les compositions destinées à la lutte contre les parasites des végétaux, les parasites des locaux et les parasites des animaux à sang chaud, caractérisées en ce qu'elles renferment comme principe actif au moins un des produits définis à l'une quelconque des revendications 1 à 5.

13. Les compositions insecticides renfermant comme principe actif au moins l'un des produits définis à l'une quelconque des revendications 1 à 5.

14. Les compositions acaricides renfermant comme principe actif au moins l'un des produits définis à l'une quelconque des revendications 1 à 5.

15. Les compositions nématicides renfermant comme principe actif au moins l'un des produits définis à l'une quelconque des revendications 1 à 5.

16. Les compositions acaricides renfermant comme principe actif l'un au moins des produits définis à l'une quelconque des revendications 1 à 5, caractérisées en ce qu'elles sont utilisées dans la lutte contre les parasites des animaux à sang chaud, notamment contre les tiques et les gales.

17. Les compositions destinées à l'alimentation animale renfermant comme principe actif au moins l'un des produits définis à l'une quelconque des revendications 1 à 5.

18. Associations douées d'acitivité insecticide, acaricide ou nématicide, caractérisées en ce qu'elles contiennent comme matière active, d'une part un au moins des composés de formule generale (I) et d'autre part, un au moins des esters pyréthrinoïdes choisis dans le groupe constitué par les ester d'alléthrolones, d'alcool 3,4,5,6-tétrahydrophtalimido méthylique, d'alcool 5-benzyl 3-furyl méthylique, d'alcool 3-phénoxy benzylique et d'alcools α -cyano 3-phénoxy benzyliques des acides chrysanthémiques, par les esters d'alcool 5-benzyl 3-furyl méthyliques des acides 2,2-diméthyl 3-(2-oxo 3-tétrahydrothiophénylidène méthyl) cyclopropane 1-carboxyliques, par les esters d'alcool 3-phénoxy benzylique et d'alcools α-cyano 3-phénoxy benzyliques des acides 2,2-diméthyl 3-(2,2-dichlorovinyl) cyclopropane 1-carboxyliques, par les esters d'alcools α-cyano 3-phénoxy benzyliques d'acides 2,2-diméthyl 3-(2,2-dibromovinyl) cyclopropane 1-carboxyliques, par les esters d'alcool 3-phénoxy benzylique des acides 2-parachloro-phényl 2-isopropyl acétiques, par les esters d'alléthrolones, d'alcool 3,4,5,6-tétrahydrophtalimido méthylique, d'alcool 5-benzyl 3-furyl méthylique, d'alcool 3-phénoxy benzylique et d'alcools α-cyano 3-phénoxy benzyliques des acides 2,2-diméthyl 3-(1,2,2,2-tétrahaloéthyl) cyclopropane 1-carboxyliques dans lesquels "halo" représente un atome de fluor, de chlore ou de brome, étant entendu que les copules acides et alcools des esters pyrethrinoïdes ci-dessus peuvent exister sous toutes leurs formes stéréoisomères possibles.

19. Les compositions selon l'une quelconque des revendications 12 à 16, caractérisées en ce qu'elles renferment en outre un synergiste des pyréthrinoïdes.

**Patentansprüche**

1. In sämtlichen ihrer stereoisomeren Formen oder in Form von Gemischen der Stereoisomeren die Verbindungen der allgemeinen Formel

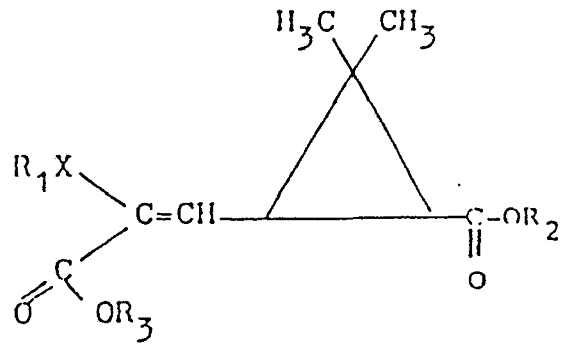

(I)

worin X ein Sauerstoff- oder Schwefelatom oder eine Sulfoxidgruppe bedeutet und

entweder $R_1$ und $R_3$, die gleich oder verschieden sind, gesättigte oder ungesättigte, lineare, verzweigte oder cyclisierte Alkyireste darstellen, deren Kette durch ein oder mehrere Heteroatome unterbrochen oder durch ein oder mehrere Halogenatome substituiert sein kann,

oder $R_1$ und $R_3$, die gleich oder verschieden sind, einen Aryloder Aralkylrest mit 6 bis 18 Kohlenstoffatomen bedeuten, und $R_2$ den Rest eines Alkohols $R_2OH$, der bei der Synthese der Pyrethrinoide verwendet wird, bedeutet.

2. Die Verbindungen der Formel I gemäß Anspruch 1, worin X ein Sauerstoffatom bedeutet.

3. Die Verbindungen der Formel I gemäß Anspruch 1, worin X ein Schwefelatom bedeutet.

4. Die Verbindungen der Formel 1 gemäß einem der Ansprüche 1, 2 oder 3, worin $R_2$ einen der folgenden Reste darstellt

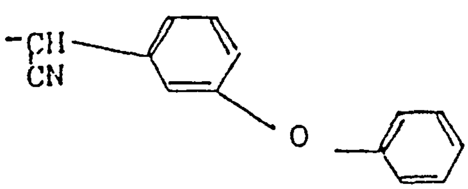

,

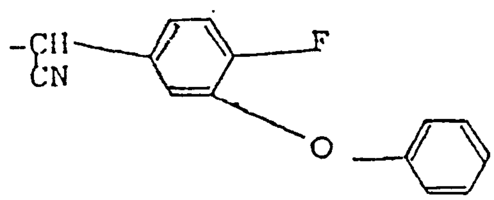

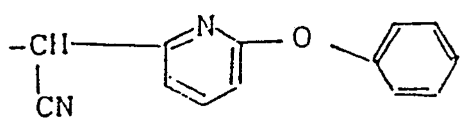

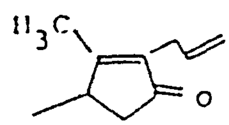

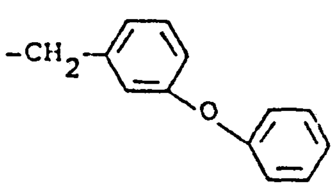

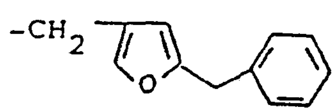

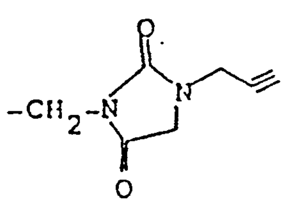

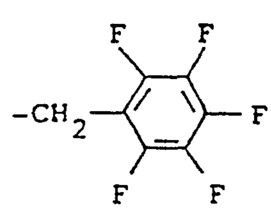

5. Die produkte der Formel I gemäß Anspruch 1 mit den folgenden Bezeichnungen:
- (S)-Cyano-1-(3-phenoxyphenyl)-methyl-1R, trans-3-(ΔZ-2-methoxy-2-methoxycarbonylethenyl) -2,2-dimethylcyclopropancarboxylat;
- (S)-Cyano-1-(3-phenoxyphenyl)-methyl-1R,trans-3-(ΔZ-2-ethoxy-2-ethoxycarbonylethenyl)-2,2-dimethylcyclopropancarboxylat;
- 3'-Allyl-2'-methyl-4'-oxo-2'-cyclopenten-1'-yl-1R,trans-3-(ΔZ-2-methoxy-2-methoxycarbonylethenyl)-2,2-dimethylcyclopropancarboxylat;
- 3'-Allyl-2'-methyl-4'-oxo-2'-cyclopenten-1'-yl-1R,trans-3-(ΔE-2-methoxy-2-methoxycarbonylethenyl)-2,2-dimethylcyclopropancarboxylat;
- [1-(3-Propin-2-yl)-2,5-dioxoimidazolidinyl]-methyl-1R,trans-3-(ΔZ-2-methoxy-2-methoxycarbonylethenyl)-2,2-dimethylcyclo-propancarboxylat;
- (S)-Cyano-1-(3-phenoxy-4-fluorphenyl)-methyl-1R,trans-3-[(ΔZ oderΔE)-2-methoxy-2-methoxycarbonylethenyl] -2,2-dimethylcyclopropancarboxylat;
- (RS)-Cyano-6'-phenoxy-2'-pyridylmethyl-1R,trans-3-[(ΔZ oder ΔE)-2-methoxy-2-methoxyethenyl]-2,2-dimethylcyclopropancarboxylat;
- (S)-Cyano-1-(3-phenoxyphenyl)-methyl-1R,trans-3-(ΔZ-2-methoxy-2-ethoxycarbonylethenyl) -2,2-dimethylcyclopropancarboxylat;
- (S)-Cyano-1-(3-phenoxyphenyl)-methyl-1R,trans-3-(ΔZ-2-methoxy-2-methoxycarbonylethenyl) -2,2-dimethylcyclopropancarboxylat;
- (S)-Cyano-1-(3-phenoxyphenyl)-methyl-1R,cis-3-(ΔZ-2-methoxy-2-methoxycarbonylethenyl)-2,2-dimethylcyclopropancarboxylat;
- (S)-Cyano-1-(3-phenoxyphenyl)-methyl-1R,cis-3-(ΔZ-2-methyl-sulfinyl-2-methoxycarbonylethenyl)-2,2-dimethylcyclopropancarboxylat (Stereoisomere A und B);
- (S)-Cyano-1-(3-phenoxyphenyl)-methyl-1R,trans-3-(ΔE-2-tert.-but-oxy-2-methoxycarbonylethenyl)-2,2-dimethylcyclopropancarboxylat;

6. Verfahren zur Herstellung der Verbindungen der allgemeinen Formel (I) gemäß Anspruch 1, dadurch gekennzeichnet, daß man eine Verbindung der Formel (II)

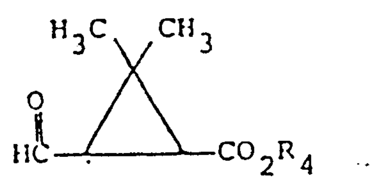

(II)

worin $R_4$ ein Wasserstoffatom oder den Rest eines leicht spaltbaren Alkohols bedeutet, die, wenn $R_4$ ein Wasserstoffatom darstellt und die Konfiguration die cis-Konfiguration ist, in der cyclisierten Form

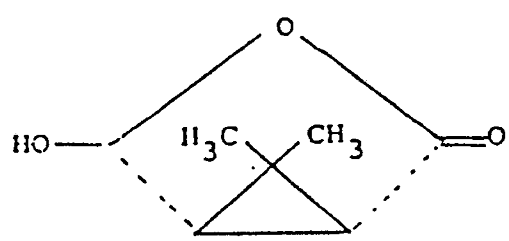

vorliegen kann mit einer Verbindung der Formel (III)

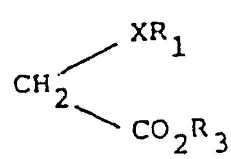

(III)

worin X, $R_1$ und $R_3$ wie in Anspruch 1 definiert sind, umsetzt, um eine Verbindung der Formel (IV)

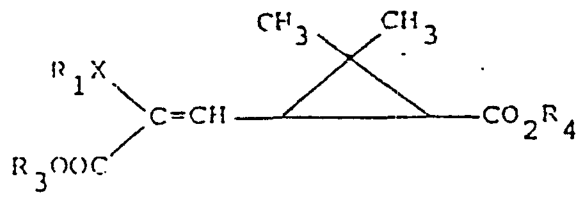

(IV)

zu erhalten, die man, wenn $R_4$ den Rest eines leicht spaltbaren Esters darstellt, der Einwirkung eines Hydrolysemittels unterzieht, um die Verbindung der Formel (IV) zu erhalten, worin $R_4$ ein Wasserstoffatom bedeutet, danach die Verbindung der Formel (IV), worin $R_4$ ein Wasserstoffatom bedeutet, der Einwirkung eines Alkohols $R_2OH$ oder derjenigen eines seiner Derivate unterzieht, um die entsprechende Verbindung der Formel (I) zu erhalten.

7. Abänderung des Verfahrens gemäß Anspruch 6, dadurch gekennzeichnet, daß man zur Herstellung der Verbindung der Formel (IV) die Verbindung der Formel (II) der Einwirkung einer Verbindung der Formel (V)

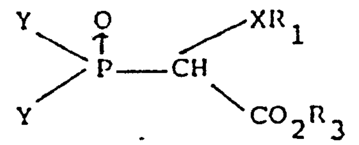

(V)

unterzieht, worin X, $R_1$ und $R_3$ die in Anspruch 1 angegebene Bedeutung besitzen, und Y einen Alkoxy- oder Arylrest mit bis zu 8 Kohlenstoffatomen bedeutet.

8. Abänderung des Verfahrens gemäß Anspruch 6 oder 7 zur Herstellung der Verbindungen der Formel (I), worin X eine Sulfoxidgruppe bedeutet, dadurch gekennzeichnet, daß man von einer Verbindung der Formel (III) oder (V) ausgeht, worin X ein Schwefelatom bedeutet, und daß man die Oxidation an der so erhaltenen Verbindung der Formel (I), worin X ein Schwefelatom darstellt, durchführt.

9. Als neue, industrielle Produkte, die Säuren der allgemeinen Formel (IV')

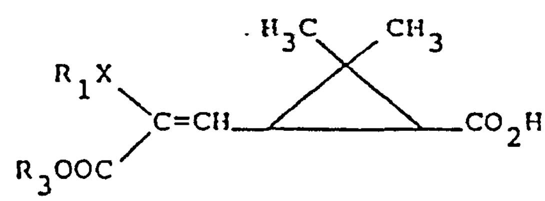

(IV')

worin $R_1$, $R_3$ und X die in Anspruch 1 angegebenen Bedeutungen besitzen.

10. Als Produkte gemäß Anspruch 9, die Säuren der Formel (IV') mit den folgenden Bezeichnungen:
- die 1R,trans-3-(ΔE-2-Methoxy-2-methoxycarbonylethenyl)-2,2-dimethylcyclopropancarbonsäure und
- die 1R,trans-3-(ΔZ-2-Methoxy-2-methoxycarbonylethenyl)-2,2-dimethylcyclopropancarbonsäure;
- die 1R,trans-3-(ΔZ-2-Methylthio-2-ethoxycarbonylethenyl)-2,2-dimethylcyclopropancarbonsäure;
- die 1R,trans-3-(ΔZ-oderΔE-2-Phenylthio-2-ethoxycarbonylethenyl) -2,2-dimethylcyclopropancarbonsäure;
- die 1R,trans-3-(ΔE-2-Ethoxy-2-ethoxycarbonylethenyl)-2,2-dimethylcyclopropancarbonsäure oder
- die 1R,trans-3-(ΔZ-2-Ethoxy-2-ethoxycarbonylethenyl) -2,2-dimethylcyclopropancarbonsäure;
- die 1R,cis-3-(ΔZ-2-Phenylthio-2-ethoxycarbonylethenyl)-2,2-dimethylcyclopropancarbonsäure;
- die 1R,cis-3-(ΔE-2-Methoxy-2-methoxycarbonylethenyl) -2,2-dimethylcyclopropancarbonsäure und die entsprechende 1R,cis-ΔZ-Säure;
- die 1R,cis-3-(ΔZ-2-Methylthio-2-methoxycarbonylethenyl)-2,2-dimethylcyclopropancarbonsäure und die entsprechendeΔE-Säure;
- die 1R,trans-3-(ΔE-2-Methoxy-2-tert.-butoxycarbonylethenyl)-2,2-dimethylcyclopropancarbonsäure und die entsprechende ΔZ-Säure;

11. Verwendung der Verbindungen der Formel (I) gemäß einem der Ansprüche 1 bis 5 zur Bekämpfung von Parasiten der Pflanzen, Parasiten von Räumlichkeiten und Parasiten warmblütiger Tiere.

12. Zusammensetzungen für die Bekämpfung von Parasiten der Pflanzen, Parasiten von Räumlichkeiten und Parasiten warmblütiger Tiere, dadurch gekennzeichnet, daß sie als Wirkstoff zumindest eines der Produkte gemäß einem der Ansprüche 1 bis 5 enthalten.

13. Insektizide Zusammensetzungen, enthaltend als Wirkstoff zumindest eines der Produkte gemäß einem der Ansprüche 1 bis 5.

14. Akarizide Zusammensetzungen, enthaltend als Wirkstoff zumindest eines der Produkte gemäß einem der Ansprüche 1 bis 5.

15. Nematizide Zusammensetzungen, enthaltend als Wirkstoff zumindest eines der Produkte gemäß einem der Ansprüche 1 bis 5.

16. Akarizide Zusammensetzungen, enthaltend als Wirkstoff zumindest eines der Produkte gemäß einem der Ansprüche 1 bis 5, dadurch gekennzeichnet, daß sie zur Bekämpfung von Parasiten warmblütiger Tiere, insbesondere von Zecken und Krätzmilben verwendet werden.

17. Zusammensetzungen für die tierische Ernährung, enthaltend als Wirkstoff zumindest eines der Produkte gemäß einem der Ansprüche 1 bis 5.

18. Mit insektizider, akarizider oder nematizider Aktivität ausgestattete Assoziationen, dadurch gekennzeichnet, daß sie als Wirkstoff einesteils zumindest eine der Verbindungen der allgemeinen Formel (I) und anderenteils zumindest einen der Pyrethrinoidester enthalten, ausgewählt unter den Estern der Allethrolone, des 3,4,5,6-Tetrahydrophthalimidomethylalkohols, des 5-Benzyl-3-furylmethylalkohols, des 3-Phenoxybenzylalkohols und der α-Cyano-3-Phenoxybenzylalkohole der Chrysanthemumsäuren, unter den Estern des 5-Benzyl-3-furylmethylalkohols der 2,2-Dimethyl-3-(2-oxo-3-tetrahydrothiophenylidenmethyl)-cyclo-propan-1-carbonsäuren, unter den Estern des 3-Phenoxybenzylalkohols und der α-Cyano-3-phenoxybenzylalkohole der 2,2-Dimethyl-3-(2,2-dichlorvinyl)-cyclopropan-1-carbonsäuren, unter den Estern der α-Cyano-3-phenoxybenzylalkohole der 2,2-Dimethyl-3-(2,2-dibromvinyl)-cyclopropan-1-carbonsäuren, unter den Estern des 3-Phenoxybenzylalkohols der 2-p-Chlorphenyl-2-isopropyl-essigsäuren, unter den Estern der Allethrolone, des 3,4,5,6-Tetrahydrophthalimidomethylalkohols, des 5-Benzyl-3-furylmethyl-alkohols, des 3-Phenoxybenzylalkohols und der α-Cyano-3-phenoxy-benzylalkohole der 2,2-Dimethyl-3-(1,2,2,2-tetrahaloethyl)-cyclopropan-1-carbonsäuren, worin "halo" ein Fluor-, Chlor- oder Bromatom bedeutet, wobei die Säure- und Alkoholverknüpfungskomponenten der vorstehenden Pyrethrinoidester in sämtlichen ihrer möglichen stereoisomeren Formen vorliegen können.

19. Zusammensetzungen gemäß einem der Ansprüche 12 bis 16, dadurch gekennzeichnet, daß sie zusätzlich einen Synergisten der Pyrethrinoide enthalten.

## Claims

1. In all their stereoisomeric forms or in the form of mixtures of stereoisomeres, the compounds with the general formula (I):

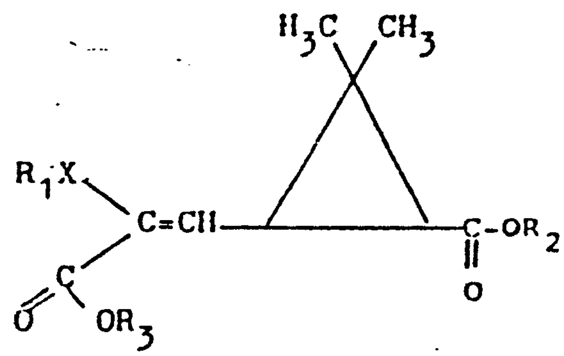

(I)

in which formula X represents an oxygen or sulphur atom, a sulphoxide group, and

either $R_1$ and $R_3$ being identical or different represent linear, branched or cyclised alkyl radicals, saturated or unsaturated, and of which the chain can be interrupted by one or more hetero-atoms, or substituted by one or more halogen atoms

or $R_1$ and $R_3$ being identical or different represent an aryl or aralkyl radical containing from 6 to 18 carbon atoms and $R_2$ represents the residue of an $R_2OH$ alcohol used in the synthesis of pyrethrinoids.

2. Compounds with the formula I according to claim 1, in which X represents an oxygen atom.

3. Compounds with the formula I according to claim 1, in which X represents a sulphur atom.

4. Compounds with the formula I according to any one of the claims 1, 2 or 3, in which $R_2$ represents one of the following radicals.

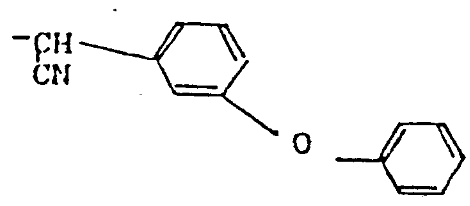

,

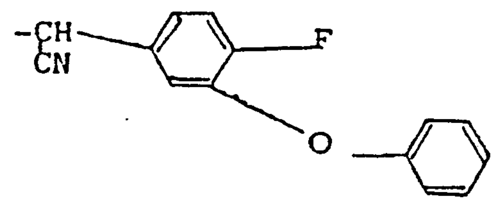

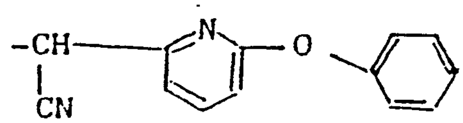

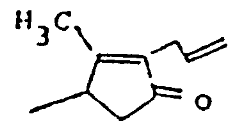

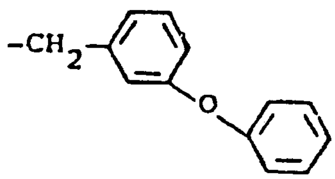

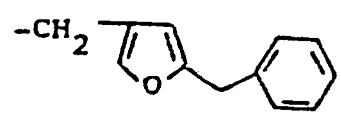

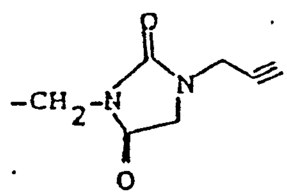

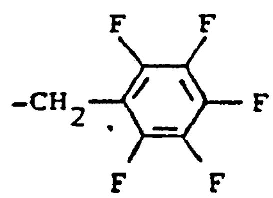

5. The products answering to the formula (I) of claim 1 the names of which follow:

- 1R,trans 3-(ΔZ 2-methoxy-2-methoxycarbonylethenyl)-2,2-dimethylcyclopropane carboxylate of (S) -cyano 1-(3-phenoxyphenyl)methyl;
- 1R,trans 3-(ΔZ 2-ethoxy-2-ethoxycarbonylethenyl)-2,2-dimethylcyclopropane carboxylate of (S) -cyano-1-(3-phenoxyphenyl methyl;
- 1R,trans 3-(ΔZ 2-methoxy-2-methoxycarbonylethenyl)-2,2-dimethylcyclopropane carboxylate of 3'-allyl-2'-methyl,4'-oxo-2'-cyclopenten-1'-yl;
- 1R,trans 3-(ΔE 2-methoxy-2-methoxycarbonylethenyl)-2,2-dimethylcyclopropane carboxylate of 3'-allyl-2'-methyl-4'-oxo-2'-cyclopenten-1'-yl;
- 1R,trans 3,(ΔZ 2-methoxy-2-methoxycarbonylethenyl)-2,2-dimethylcyclopropane carboxylate of [1-(3-propyn-2-yl)-2,5-dioxoimidazolidinyl]methyl;
- 1R,trans 3-[(Δ Z or ΔE)-2-methoxy-2-methoxycarbonyleth-enyl]-2,2-dimethylcyclopropane carboxylate of (S) cyano-1,(3-phenoxy-4-fluorophenyl)methyl;
- 1R,trans 3-[(Δ Z or ΔE)-2-methoxy-2-methoxycarbonylethenyl]-2,2-dimethylcyclopropane carboxylate of (RS) cyano 6'-phenoxy-2'-pyridyl methyl;
- 1R,trans 3-(ΔZ 2-methoxy-2-ethoxycarbonylethenyl)-2,2-dimethylcyclopropane carboxylate of (S) cyano-1-(3-phenoxyphenyl)methyl;
- 1R,trans 3-(ΔZ 2-methoxy-2-methoxycarbonylethenyl)-2,2-dimethylcyclopropane carboxylate of (S) cyano 1-(3-phenoxyphenyl)methyl;
- 1R,cis 3-(ΔZ 2-methoxy-2-methoxycarbonylethenyl)-2,2-dimethylcyclopropane carboxylate of (S) cyano-1-(3-phenoxyphenyl)methyl;
- 1R,cis 3-(ΔZ 2-methylsulphinyl-2-methoxycarbonylethen-yl)-2,2-dimethylcyclopropane carboxylate of (S) cyano 1-(3-phenoxyphenyl)methyl (stereoisomers A and B);
- 1R,trans 3-(ΔE 2-tert-butoxy-2-methoxycarbonylethenyl) 2,2-dimethylcyclopropane carboxylate of (S) cyano-1-(3-phenoxyphenyl)methyl.

6. Preparation process of compounds with the general formula (I) as defined in claim 1, characterized in that a compound with the formula (II):

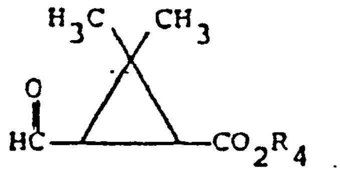

(II)

in which $R_4$ represents a hydrogen atom or the residue of an easily cleavable alcohol, able to appear in the case where $R_4$ is a hydrogen atom and the configuration is cis, in the cyclised form,

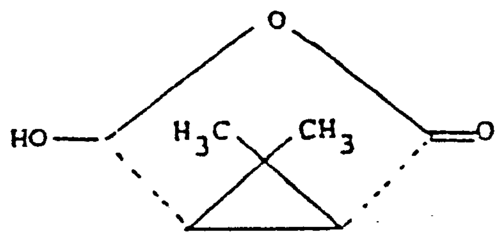

is made to react with a compound with the formula (III)

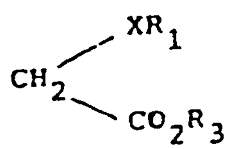

(III)

in which X, $R_1$ and $R_3$ are defined as in claim 1, so as to obtain a compound with the formula (IV):

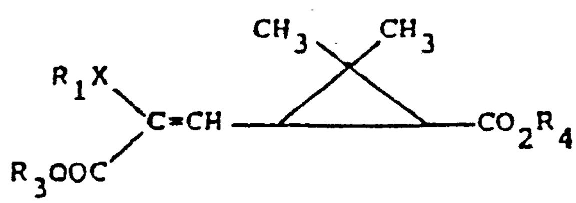

(IV)

which, in the case when $R_4$ represents an easily cleavable ester residue, is submitted to the action of a hydrolysis agent, so as to obtain the compound with the formula (IV) in which $R_4$ represents a hydrogen atom, then the compound with the formula (IV) in which $R_4$ represents a hydrogen atom is submitted to the action of a $R_2OH$ alcohol or one of its derivatives so as to obtain the corresponding compound with the formula (I).

7. Variant of the process according to claim 6, characterized in that for the preparation of the compound with the formula (IV) the compound with the formula (II) is submitted to the action of a compound with the formula (V):

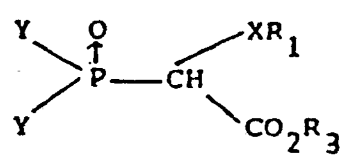

(V)

in which X, $R_1$, and $R_3$ retain the same significance as in claim 1 and Y represents an alkoxyl or aryl radical containing up to 8 carbon atoms.

8. Variant of the process according to claim 6 or 7, for the preparation of compounds with the formula (I) in which X represents a sulphoxide group, characterized in that at the start a compound with the formula (III) or (V) is used in which X represents a sulphur atom and the oxidation is carried out on the compound with the formula (I) thus obtained, in which X represents a sulphur atom.

9. As new industrial products, acids with the general formula (IV')

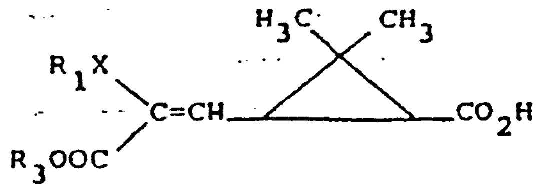

(IV')

in which formula $R_1$, $R_3$ and X retain the significances of claim 1.

10. As products as defined in claim 9, the acids with the formula (IV') the names of which follow:

- 1R,trans 3-(Δ E 2-methoxy,2-methoxy carbonyl ethenyl)-2,2-dimethylcyclopropane carboxylic acid and 1R,trans 3-(ΔZ 2-methoxy-2-methoxycarbonylethenyl)-2,2-dimethyl-cyclopropane carboxylic acid;
- 1R,trans 3-(ΔZ 2-methylthio-2-ethoxycarbonylethenyl)-2,2-dimethylcyclopropane carboxylic acid;
- 1R,trans 3,(Δ Z or Δ E 2-phenylthio-2-ethoxycarbonyl-ethenyl)-2,2-dimethylcyclopropane carboxylic acid;
- 1R,trans 3-(ΔE 2-ethoxy-2-ethoxycarbonylethenyl)-2,2-dimethylcyclopropane carboxylic acid and 1R,trans 3-(Δ Z 2-ethoxy-2,ethoxycarbonylethenyl)-2,2-dimethylcyclo-propane carboxylic acid;
- 1R,cis 3-(ΔZ 2-phenylthio-2-ethoxycarbonylethenyl)-2,2-dimethylcyclopropane carboxylic acid;
- 1R,cis 3-(ΔE 2-methoxy-2-methoxycarbonylethenyl]-2,2-dimethylcyclopropane carboxylic acid and the corresponding 1R,cis ΔZ acid;
- 1R,cis 3-(Δ Z 2-methylthio-2-methoxycarbonylethenyl)-2,2-dimethylcyclopropane carboxylic acid and the corresponding ΔE acid;
- 1R,trans 3-(ΔE 2-methoxy-2-tert-butoxycarbonylethenyl) -2,2-dimethylcyclopropane carboxylic acid and the corresponding ΔZ acid;

11. Use of the compounds with the formula I as defined in any one of the claims 1 to 5 to combat parasites of vegetation, parasites of premises and parasites of warmblooded animals.

12. Compositions intended to combat parasites of vegetation, parasites of premises and parasites of warmblooded animals, characterized in that they contain as active principle at least one of the products defined in any one of the claims 1 to 5.

13. Insecticide compositions containing as active principle at least one of the products defined in any one of the claims 1 to 5.

14. Acaricide compositions containing as active principle at least one of the products defined in any one of the claims 1 to 5.

15. Nematocide compositions containing as active principle at least one of the products defined in any one of the claims 1 to 5.

16. Acaricide compositions containing as active principle at least one of the products defined in any one of the claims 1 to 5, characterized in that they are used to combat parasites of warm-blooded animals, in particular ticks and mites.

17. Compositions intended for animal foodstuffs containing as active principle at least one of the products defined in any one of the claims 1 to 5.

18. Combinations endowed with insecticide, acaricide or nematocide activity, characterized in that the contain as active material, on the one hand at least one of the compounds with the general formula (I), and on the other hand, one at least of the pyrethrinoid esters chosen from the group constituted by the esters of allethrolones, of 3,4,5,6-tetrahydrophthalimidomethyl alcohol, of 5-benzyl-3-furyl methyl alcohol, of 3-phenoxybenzyl alcohol and of α-cyano-3-phenoxybenzyl alcohols with chrysanthemic acids, by the esters of 5-benzyl-3-furyl methyl alcohol with 2,2-dimethyl-3-(2-oxo-3-tetrahydrothiophenylidenemethyl) cyclopropane-1-carboxylic acids, by the esters of 3-phenoxybenzyl alcohol and of α-cyano-3-phenoxybenzyl alcohols with 2,2-dimethyl-3-(2,2-dichlorovinyl)cyclopropane-1-carboxylic acids, by the esters of α-cyano-3-phenoxybenzyl alcohols with 2,2-dimethyl-3-(2,2-dibromovinyl)cyclopro-pane-1-carboxylic acids, by the esters of 3-phenoxy-benzyl alcohol with 2-parachlorophenyl-2-isopropyl acetic acids, by the esters of allethrolones, of 3,4,5,6-tetrahydrophthalimidomethyl alcohol, of 5-benzyl -3-furyl methyl alcohol, of 3-phenoxybenzyl alcohol and of α-cyano-3-phenoxybenzyl alcohols with 2,2-dimethyl-3-(1,2,2,2-tetrahaloethyl)cyclopropane-1-carboxylic acids, in which "halo" represents a fluorine, chlorine or bromine atom, it being understood that the acid and alcohol copulas of the above pyrethrinoid esters can exist in all their possible stereoisomeric forms.

19. Compositions according to any one of the claims 12 to 16 characterized in that they also contain a synergist of pyrethrinoids.